(19) Europäisches Patentamt
European Patent Office
Office européen des brevets

(11) Publication number: **0 084 928**
**B1**

# (12) EUROPEAN PATENT SPECIFICATION

(45) Date of publication of patent specification: **11.09.85**

(51) Int. Cl.$^4$: **C 07 C 101/72,** C 07 C 99/00, C 07 D 317/60

(21) Application number: **83300059.9**

(22) Date of filing: **06.01.83**

## (54) Process for producing threo-3-(3,4-dihydroxyphenyl)serine.

(30) Priority: **14.01.82 JP 4501/82**
**20.04.82 JP 66491/82**
**21.04.82 JP 67963/82**
**09.06.82 JP 99786/82**
**24.09.82 JP 167058/82**

(43) Date of publication of application:
**03.08.83 Bulletin 83/31**

(45) Publication of the grant of the patent:
**11.09.85 Bulletin 85/37**

(84) Designated Contracting States:
**AT BE CH DE FR GB IT LI NL SE**

(56) References cited:
**EP-A-0 024 210**
**FR-A-2 241 302**
**US-A-4 051 169**

**CHEMICAL ABSTRACTS, vol. 91, no. 7, 13th August 1979, page 761, no. 57535m, Columbus, Ohio, USA**

**HELVETICA CHIMICA ACTA, vol. 58, fasc. 1, no. 19, January 1975, pages 147-162, B. HEGEDÜS et al.: "Die Synthese von (-)- bzw. (+)-threo-3-(3,4-Dihydroxyphenyl)-serin und (-)- bzw. (+)-erythro-3-(3,4-Dihydroxyphenyl)-serin"**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Ohashi, Naohito**
**2-406, Ryodocho-4-chome**
**Nishinomiya-shi (JP)**
Inventor: **Nagata, Shoji**
**10-4, Sonehigashinocho-2-chome**
**Toyonaka-shi (JP)**
Inventor: **Ishizumi, Kikuo**
**16-10, Ueonishi-2-chome**
**Toyonaka-shi (JP)**
Inventor: **Maeshima, Kaoru**
**7-4, Nakakonoike-1-chome**
**Higashiosaka-shi (JP)**

(74) Representative: **Harrison, David Christopher et al**
**MEWBURN ELLIS & CO 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to a process for producing racemic or optically active threo-3-(3,4-dihydroxyphenyl)serine represented by the formula,

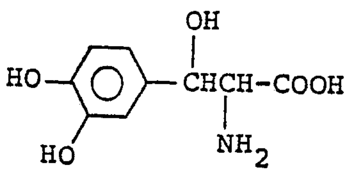

[1].

It relates also to racemic or optically active threo-N-carbobenzoxy*-3-(3,4-methylenedioxyphenyl)serine represented by the formula

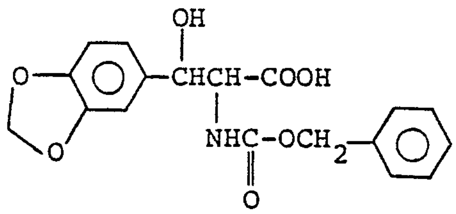

[3"]

which is useful in producing the compound of formula [1], and to a process for producing same.

* Or "N-benzyloxycarbonyl".

The optically active threo-3-(3,4-dihydroxyphenyl)serine (hereinafter referred to briefly as DOPS) is known to be a pharmaceutical useful as a remedy for peripheral orthostatic hypotension [Japanese Patent Application "Kokai" (Laid-open) No. 104,815/81] or as an antidepressant [Japanese Patent Application "Kokai" (Laid-open) No. 20,747/80].

Known processes for the preparation of optically active DOPS of formula [1] include those disclosed in Japanese Patent Application "Kokai" (Laid-open) Nos. 49,252/75 (Method A), 36,233/79 (Method B), 29,551/81 (Method C), and 32,540/76 (Method D).

In the methods A, B and C, there is employed, as the starting material, a protocatechualdehyde of the formula,

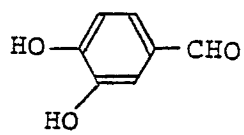

This aldehyde may be obtained from vanilline, veratraldehyde or piperonal wherein its catechol moiety is protected each by a methyl group, two methyl groups or a methylene group. Thus, the procedure of these methods comprises removing the methyl or methylene group from vanilline, veratraldehyde or piperonal to yield protocatechualdehyde, protecting again the catechol moiety by benzyl groups to form a benzaldehyde derivative of the formula

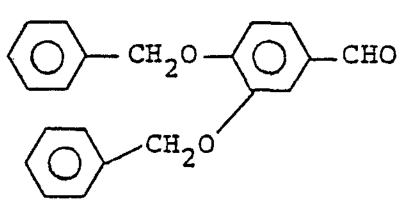

then condensing the resulting derivative with glycine to yield a mixture of threo- and erythro-3-(3,4-dibenzyloxyphenyl)serines of the formula

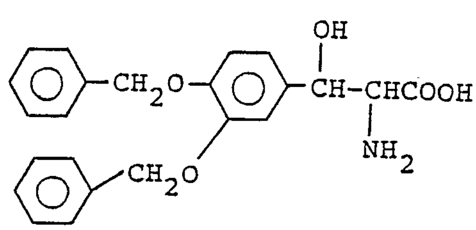

(the formula represents both threo and erythro forms), converting, if necessary, the mixture to appropriate derivative, and separating the mixture into the threo and erythro forms, subjecting each form to optical resolution, and finally removing the protective group to yield an optically active DOPS.

The method D comprises first preparing racemic DOPS from the threo-3-(3,4-dibenzyloxyphenyl)serine obtained as described above, then reaching the resulting racemate with carbobenzoxy chloride (Benzyloxycarbonyl chloride) to yield racemic threo-N-carbobenzoxy-3-(3,4-dihydroxyphenyl)serine, subjecting the resulting protected racemate to optical resolution, and finally removing the protective group to give an optically active DOPS.

As described above, all of the known methods for the preparation of optically active DOPS have in common a disadvantage of adopting a round-about way of synthesis involving replacement of the protective group in catechol moiety of the benzaldehyde derivative used as the starting material. Method D has a further disadvantage in the complicated procedure of preparing racemic DOPS at first, then reintroducing a protective group which is finally removed. In all of the known methods, an optically active DOPS is obtained by first protecting the functional group of DOPS, and subjecting the protected DOPS to optical resolution using an optically active amine as resolving agent to yield an optically active DOPS derivative which is then converted to an optically active DOPS. There is no known case where the racemic DOPS is directly resolved into optically active isomers.

Under the circumstances, we made an extensive study on the process for producing optically active DOPS without the need of the replacement of a protective group in the catechol moiety. As a result, it was found that optically active DOPS is readily produced by using as the intermediate racemic or optically active threo-N-carbobenzoxy-3-(3,4-methylenedioxyphenyl)serine of the abovementioned formula [3″] which is a novel compound not found in the literature. Furthermore, as a result of an extensive study on the method for producing optically active DOPS directly from racemic DOPS by optical resolution, it was found that the racemic DOPS is a racemic mixture susceptible to the optical resolution by preferential crystallization, without the need of prior conversion into a derivative. Thus the present invention provides a process for producing threo-3-(3,4-dihydroxyphenyl)serine of the abovementioned formula [1], which process comprises treating threo-3-(3,4-methylenedioxyphenyl)serine or an N-carbobenzoxy derivative thereof represented by the formula

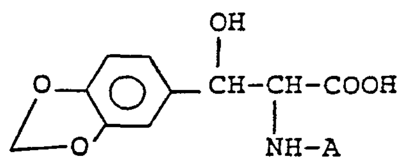 [3],

wherein A represents a hydrogen atom or a carbobenzoxy group, with a Lewis acid to form threo-3-(3,4-dihydroxyphenyl)serine or an N-carbobenzoxy derivative thereof represented by the formula

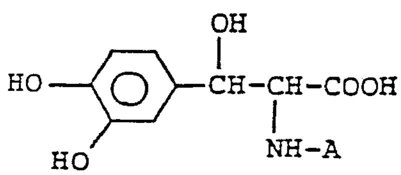 [2],

wherein A is as defined above, and, if A is a carbobenzoxy group, catalytically reducing the resulting compound of formula [2].

Certain aspects of the present invention rely upon one or more of the following facts which we have found:

1. Racemic threo-3-(3,4-methylenedioxyphenyl)serine acetate can be obtained without any special separation treatment by the condensation of glycine with piperonal.

2. When racemic or optically active threo-N-carbobenzoxy-3-(3,4-methylenedioxyphenyl)serine is treated with a Lewis acid, only the methylene portion of the methylenedioxy group can be removed in spite of the presence of carboxyl, carbamate and hydroxyl groups in the same molecule.

3. DL-threo-N-Carbobenzoxy-3-(3,4-methylenedioxyphenyl)serine and DL-threo-N-carbobenzoxy-3-(3,4-dihydroxyphenyl)serine can be optically resolved to yield an optically active (D- or L-) form of the corresponding compounds by using as the resolving agent an optically active amine which is suitable for industrial use.

4. The racemic DOPS is directly resolvable by the preferential crystallization technique without prior conversion to a derivative.

Accordingly, the invention provides an economical process for the production of optically active DOPS.

On the other hand, although a method is known to form 3-(3,4-methylenedioxyphenyl)serine from glycine and piperonal ["Yakugaku Zasshi" (Journal of the Pharmaceutical Society, Japan), *67*, 218 (1947);

Can. J. Chem., *42,* 1901 (1964)], no information has been available prior to the applicant's invention on the threo and erythro stereoisomers of the serine compound. To our knowledge the threo stereoisomer has been disclosed and isolated for the first time by us.

Further, since the threo-3-(3,4-dibenzyloxyphenyl)serine conventionally used as the intermediate in the production of racemic and optically active DOPS is obtained as a mixture of threo and erythro stereoisomers by the reaction between glycine and a benzaldehyde derivative as stated above, it is necessary to separate the mixture into threo and erythro stereoisomers [J. Am. Chem. Soc., *76,* 1322 (1954); Japanese Patent Application "Kokai" (Laid-open) Nos. 49,252/75 and 19,931/79]. In contrast, by a process embodying this invention, the threo-3-(3,4-methylenedioxyphenyl)serine is readily obtained without the need of special separation procedure.

We believe that racemic threo-N-carbobenzoxy-3-(3,4-methylenedioxyphenyl)serine is a novel compound obtained by the reaction of racemic threo-3-(3,4-methylenedioxyphenyl)serine with carbobenzoxy chloride. An optically active threo-3-(3,4-methylenedioxyphenyl)serine can be obtained by reacting said racemic N-carbobenzoxy derivative with one of the optically active amines including ephedrine, quinidine, quinine and 2-amino-1,1-diphenylpropanol to yield a mixture of amine salts of D- and L-threo-N-carbobenzoxy-3-(3,4-methylenedioxyphenyl)serine, separating the mixture by taking advantage of the solubility difference into the amine salt of D-threo-N-carbobenzoxy-3-(3,4-methylenedioxyphenyl)serine and the amine salt of L-threo-N-carbobenzoxy-3-(3,4-methylenedioxyphenyl)serine, and finally reacting each amine salt with an acid.

There is no known method for converting a racemic or optically active threo-N-carbobenzoxy-3-(3,4-methylenedioxyphenyl)serine into corresponding threo-N-carbobenzoxy-3-(3,4-dihydroxyphenyl)serine. Various methods are known for the removal of a methylene group from a compound having a methylenedioxy group to form a catechol group. Furthermore, for a compound having amino and carboxyl groups in addition to the methylenedioxy group, there is found a report about the case where 3-(3,4-methylenedioxyphenyl)alanine or an N-acetyl derivative thereof is treated with hydroiodic acid and acetic anhydride in the presence of red phosphorus to obtain 3-(3,4-dihydroxyphenyl)alanine [Chem. Pharm. Bull., *10,* 693 (1962)]. However, we have found no report of a compound having hydroxyl, carbamate, carboxyl, and methylenedioxy groups all together. We therefore, conducted an extensive study on the conversion of a racemic or optically active threo-N-carbobenzoxy-3-(3,4-methylenedioxyphenyl)serine having hydroxyl, carbamate, and carboxyl groups in addition to the methylenedioxy group into corresponding racemic or optically active threo-N-carbobenzoxy-3-(3,4-dihydroxyphenyl)serine. It was found, as a result, that it is possible to achieve the conversion by the treatment with a Lewis acid under mild conditions. The addition of a mercaptan along with the Lewis acid is favorable to the reaction.

The method for the formation of an optically active threo-N-carbobenzoxy-3-(3,4-dihydroxyphenyl)serine from racemic threo-N-carbobenzoxy-3-(3,4-dihydroxyphenyl)serine by the optical resolution technique is known [Japanese Patent Application "Kokai" (Laid-open) No. 32,540/76]. In the known method, an expensive resolving agent, quinone is used. We investigated the resolution with a resolving agent available at low cost or one which is easily prepared. As a result, it was found that the optical resolution is possible by using as the resolving agent cinchonidine, brucine or ephedrine, all of which are available at a lower cost than quinine, or 2-amino-1,1-diphenylpropanol which is easily prepared [Japanese Patent Application "Kokai" (Laid-open) No. 141666/79]. It is thus possible to obtain an optically active threo-N-carbobenzoxy-3-(3,4-dihydroxyphenyl)serine by reacting racemic threo-N-carbobenzoxy-3-(3,4-dihydroxyphenyl)serine with an optically active amine selected from cinchonidine, brucine, ephedrine, and 2-amino-1,1-diphenylpropanol to yield a mixture of amine salts of D- and L-threo-N-carbobenzoxy-3-(3,4-dihydroxyphenyl)serine, separating the mixture by taking advantage of the solubility difference into the amine salt of D-threo-N-carbobenzoxy-3-(3,4-dihydroxyphenyl)serine and the amine salt of L-threo-N-carbobenzoxy-3-(3,4-dihydroxyphenyl)serine, and finally reacting each amine salt with an acid.

The conversion of an optically active threo-N-carbobenzoxy-3-(3,4-dihydroxyphenyl)serine into optically active DOPS is achieved under the reaction conditions commonly employed in removing the carbobenzoxy group in the reactions of amino acids or peptides.

The resolution of racemic DOPS into optically active DOPS isomers is described below.

Among the methods for the resolution of a racemate into optically active isomers, the so-called preferential crystallization is based on the well-known principle that crystals of one of the optical isomers are seeded into a supersaturated solution of the racemate to collect the same optical isomers as that of the seed. However, it is said that the above principle is applicable only to the case where the racemate forms a racemic mixture (conglomerate), but not to the case of a racemic compound (molecular compound). It is unknown and beyond anticipation, however, which particular type of compound may form a racemic mixture or may be suitable for the application of said principle. It may be concluded, therefore, that the racemate forms a racemic mixture only in rather unusual cases which may be found with much difficulty. Moreover, a racemate is rarely resolvable as such by the preferential crystallization [A. Collect et al., Chem., Rev. *80,* 215 (1980)]. After a great many trials of optical resolution of racemic DOPS, we found that the racemic DOPS forms a racemic mixture which, as such, meets the necessary conditions for the resolution by preferential crystallization. This discovery contributed to the accomplishment of this invention. According to an embodiment of this invention, crystals of an optically active DOPS are placed in a supersaturated solution of racemic DOPS to crystallize preferentially the same optical isomer as that of the seed

crystals and the crystallized isomer is collected to obtain optically active DOPS. Either L-DOPS or D-DOPS may be obtained by selecting the type of optical activity of the seed crystals.

Processes embodying the present invention are schematically represented in the following Figure.

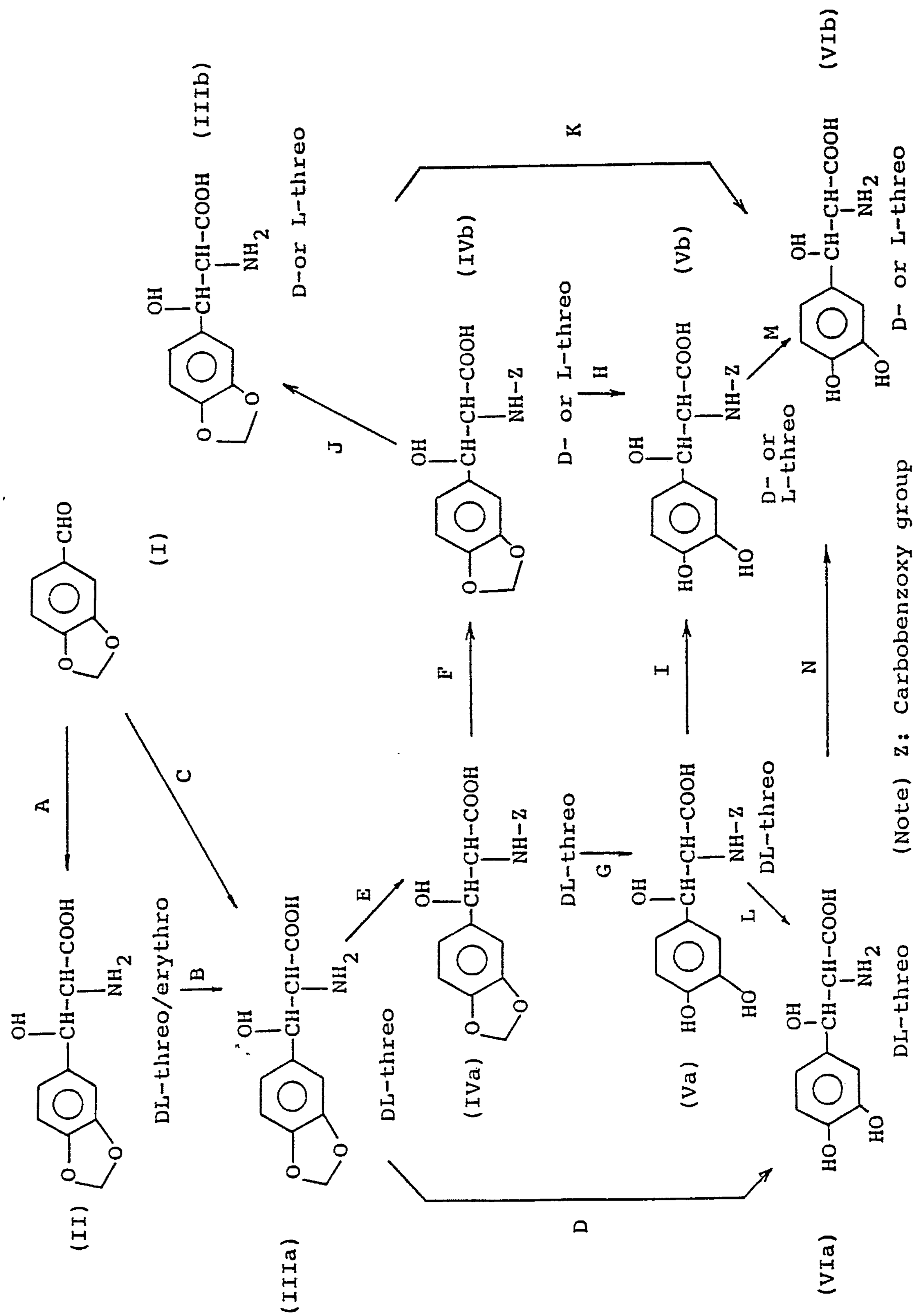


(Note) Z: Carbobenzoxy group

Methods by which each of the reaction steps of the processes illustrated in the Figure may be carried out are described below in detail.

Step A (Compound I → II):

Compound II, threo/erythro-3-(3,4-methylenedioxyphenyl)serine, is obtained in the form of crystalline acetate by the reaction of 1 mole of glycine, 2 moles of piperonal (I) and 2 moles of a base such as sodium hydroxide or potassium hydroxide in a solvent such as methanol, ethanol or the like, at a temperature of 50°—70°C for 0.5—2 hr and subsequent treatment of the reaction product with aqueous acetic acid.

Step B (Compound II → IIIa):

In this step, crystalline threo-3-(3,4-methylenedioxyphenyl)serine (IIIa) is obtained from the acetate of threo/erythro-3-(3,4-methylenedioxyphenyl)serine, a mixture of threo and erythro forms, by recrystallizing from water or by suspending the acetate in water and collecting the suspended matter by filtration, whereby the separation of threo form from erythro form takes place. In this step, there is obtained free amino acid from the acetate with the elimination of acetic acid moiety. Although no special restriction is posed on the ratio of threo form to erythro form, it is preferred that the mixture contains excess threo form. The recrystallization is performed in the usual manner. When the separation is to be effected by suspending the acetate in water followed by filtration, the threo form (IIIa) is obtained by adding water in an amount of about 5 to 15 times the quantity of acetate, stirring the resulting suspension at 20° to 60°C, then cooling to 0° to 30°C, and filtering. The stirring in water can be performed at room temperature or thereabout.

Step C (Compound I → IIIa):

The acetate of racemic threo-3-(3,4-methylenedioxyphenyl)serine (IIIa) is also obtained directly by the condensation of glycine and piperonal in the presence of a base and subsequent addition of acetic acid to the reaction mixture. Piperonal and the base are used in an amount of preferably 2 to 3 moles and 1.5 to 2.5 moles, respectively, per 1 mole of glycine. Suitable bases are inorganic ones such as sodium hydroxide, potassium hydroxide, sodium carbonate, potassium carbonate, and the like. The reaction media are alcoholic solvents such as methanol, ethanol, and isopropyl alcohol, ether-type solvents such as tetrahydrofuran and dioxane, and mixtures of these solvents with water. The condensation temperature and the reaction time are in the range of from −10° to 40°C and 5—20 hr, respectively, in order to minimize the accompanying racemic erythro-3-(3,4-methylenedioxyphenyl)serine. After completion of the condensation, the intermediate products of the reaction are decomposed by the addition of water and acetic acid. To the reaction mixture is added an organic solvent such as toluene, benzene, ethyl acetate, and diethyl ether in order to transfer the piperanol to the organic layer and to collect precipitated acetate of racemic threo-3-(3,4-methylenedioxyphenyl)serine. The amount of water to be added is twice or more times, preferably 3 to 20 times the weight of glycine and the amount of acetic acid is three or more times, preferably 5 to 30 times the weight of glycine.

In the above procedure, the threo form precipitates in the form of acetate, whereas the erythro form remains as dissolved in the medium. Although racemic 3-(3,4-methylenedioxyphenyl)serine of unknown steric configuration (threo-erythro isomerism) is already known [Yakugaku Zasshi, *67*, 218 (1947); Can. J. Chem., *42*, 1901 (1964)], the threo form has been isolated and identified for the first time by the present inventors.

Step D (Compound IIIa → VIa):

In this step, threo-3-(3,4-methylenedioxyphenyl)serine is treated with a Lewis acid to give DOPS (VIa). Examples of preferable Lewis acids are aluminum chloride, aluminum bromide, ferric chloride, stannic chloride, boron trichloride, and boron tribromide. It is also possible to use a complex of a Lewis acid and dimethyl sulfide. The amount to be used of Lewis acid is 1 to 20, preferably 2 to 10, moles per 1 mole of threo-3-(3,4-methylenedioxyphenyl)serine (IIIa). In some cases, favorable results are obtained by adding to the reactant mixture, in addition to the Lewis acid, a mercaptan such as methylmercaptan, ethylmercaptan, butylmercaptan, octylmercaptan, dodecanylmercaptan, octadecanylmercaptan or thiophenol in an amount of 1 to 4 moles per 1 mole of the Lewis acid. Any solvent which does not interfere with progress of the reaction may be used as the reaction medium. Preferable solvents include halogenated hydrocarbon-type solvents such as dichloromethane, chloroform, dichloroethane, and chlorobenzene; aromatic hydrocarbons such as toluene and benzene; esters such as ethyl acetate and butyl acetate; nitrohydrocarbons such as nitromethane, nitroethane, and nitrobenzene; and ketones such as acetone and methyl ethyl ketone. If necessary, DOPS which is produced can be recrystallized from water.

The above procedure is applicable to both the racemate and the optically active isomers.

Step E (Compound IIIa → IVa):

Racemic threo-N-carbobenzoxy-3-(3,4-methylenedioxyphenyl)serine (IVa) is obtained by so-called Schotten-Baumann reaction between racemic threo-3-(3,4-methylenedioxyphenyl)serine (IIIa) and carbobenzoxy chloride. This reaction is carried out by adding the latter to an alkaline aqueous solution (pH 7 or above) of the former. The amount by mole used of the latter is 1 to 2 times the amount of the formers. The

reaction is allowed to proceed at a reaction temperature of 0° to 30°C for several minutes to 20 hours. It is preferable to maintain pH at 7 to 10 during the reaction by the dropwise addition of an aqueous alkali solution to keep the pH at the predetermined level or by the prior addition of sodium hydrogencarbonate, borax, or the like to the reactor so as to keep the aqueous solution at pH 7—10. Alkalis for use in the alkaline aqueous solution include sodium hydrogencarbonate, sodium carbonate, potassium carbonate, sodium hydroxide, and potassium hydroxide. The reaction proceeds sufficiently in an aqueous solution. If required, there may be added aromatic hydrocarbons such as toluene and benzene; aliphatic hydrocarbons such as hexane and heptane; ethers such as diethyl ether, tetrahydrofuran, and dioxane; ketones such as acetone and methyl ethyl ketone; alkyl halide-type solvents such as chloroform, dichloromethane, and dichloroethane; esters such as ethyl acetate and butyl acetate; and mixtures of these solvents.

Step F (Compound IVa → IVb):

An optically active threo-N-carbobenzoxy-3-(3,4-methylenedioxyphenyl)serine (IVb) is obtained by reacting, in a suitable solvent, racemic threo-N-carbobenzoxy-3-(3,4-methylenedioxyphenyl)serine (IVa) with an optically active amine selected from ephedrine, quinidine, quinine, and 2-amino-1,1-diphenylpropanol to yield the salts of D- and L-threo-N-carbobenzoxy-3-(3,4-methylenedioxyphenyl)serine with the optically active amine, separating the salts into the salt of D-threo-N-carbobenzoxy-3-(3,4-methylenedioxyphenyl)serine with the optically active amine and the salt of L-threo-N-carbobenzoxy-3-(3,4-methylenedioxyphenyl)serine with the optically active amine by taking advantage of the solubility difference, and decomposing each salt with an acid.

The formation and fractionation of the amine salt are performed at 0° to 80°C, or by heating to a temperature near the boiling point of the solvent and cooling to 0°C to 30°C. The formation of the amine salt is complete within several minutes but the reaction time can be extended to several hours without causing any disturbance. The molar ratio of the optically active amine to the racemic threo-N-carbobenzoxy-3-(3,4-methylenedioxyphenyl)serine is from 0.5 to 1. Examples of solvents suitable for the formation and fractionation of the amine salts are alcohols such as methanol, ethanol, and isopropyl alcohol; ethers such as tetrahydrofuran and dioxane; acetonitrile, water, and mixtures of these solvents.

The optically active threo-N-carbobenzoxy-3-(3,4-methylenedioxyphenyl)serine is obtained by adding an aqueous acidic solution to the salt of optically active threo-N-carbobenzoxy-3-(3,4-methylenedioxyphenyl)serine with the optically active amine to decompose the salt, and extracting with an organic solvent. The acids suitable for use in the aqueous acidic solution are mineral acids such as hydrochloric acid, sulfuric acid, and phosphoric acid. The amount by mole to be used of the acid is 1 to 10 moles per 1 mole of amine salt. The organic solvents suitable for the extraction include ethyl acetate, chloroform, dichloroethane, dichloromethane, and diethyl ether.

The racemic and optically active threo-N-carbobenzoxy-3-(3,4-methylenedioxyphenyl)serine (IVa and IVb), which are novel compounds not found in the literature, have an antimicrobial activity by themselves and are useful not only as the intermediates in synthesizing DOPS, but also as pharmaceuticals by themselves.

Step G (Compound IVa → Va) and Step H (Compound IVb → Vb)

When racemic or optically active threo-N-carbobenzoxy-3-(3,4-methylenedioxyphenyl)serine (IVa and IVb) is treated with a Lewis acid in a suitable solvent, there is obtained corresponding racemic or optically active threo-N-carbobenzoxy-3-(3,4-dihydroxyphenyl)serine (Va and Vb). Examples of suitable Lewis acids are aluminum chloride, aluminum bromide, ferric chloride, stannic chloride, boron trichloride, and boron tribromide. A complex of a Lewis acid and dimethyl sulfide may also be used as a Lewis acid. The amount to be used of a Lewis acid is 1 to 20, preferably 2 to 10, moles per 1 mole of threo-N-carbobenzoxy-3-(3,4-methylenedioxyphenyl)serine. To obtain more desirable results, there may be added to the reactant mixture, in addition to a Lewis acid, a mercaptan of 1 to 20 carbon atoms such as methylmercaptan, ethylmercaptan, butylmercaptan, octylmercaptan, dodecanylmercaptan, octadecanylmercaptan and thiophenol, in an amount of 1 to 5 moles per 1 mole of Lewis acid. Although any of the solvents which do not interfere with the progress of the reaction may be used, preferable solvents are halogenated hydrocarbon-type solvents such as dichloromethane, chloroform, dichloroethane, and chlorobenzene; aromatic hydrocarbons such as toluene and benzene; esters such as ethyl acetate and butyl acetate; nitrohydrocarbons such as nitromethane, nitroethane, and nitrobenzene; ketones such as acetone and methyl ethyl ketone; pyridine, and mixtures of these solvents. The reaction is carried out at a temperature in the range of from −40°C to 80°C, preferably from −10° to 30°C. The reaction is complete within the range of from 10 minutes to 4 hours. A more extended reaction time has no adverse effect.

Step I (Compound Va → Vb):

An optically active threo-N-carbobenzoxy-3-(3,4-dihydroxyphenyl)serine (Vb) is obtained by reacting racemic threo-N-carbobenzoxy-3-(3,4-dihydroxyphenyl)serine (Va) with an optically active amine selected from cinchonidine, brucine, ephedrine, and 2-amino-1,1-diphenylpropanol to yield the amine salts of D- and L-threo-N-carbobenzoxy-3-(3,4-dihydroxyphenyl)serine, separating the salts into the amine salt of D-threo-N-carbobenzoxy-3-(3,4-dihydroxyphenyl)serine and the amine salt of L-threo-N-carbobenzoxy-3-(3,4-dihydroxyphenyl)serine by taking advantage of the solubility difference, and reacting each amine salt with

an acid. The optical resolution and the decomposition of salt described above can be carried out under the same conditions of molar ratio, solvent, temperature, and time as used in Step F, except for the optically active amine.

Steps J and K (Compound IVb → IIIb → VIb):

An optically active threo-3-(3,4-methylenedioxyphenyl)serine (IIIb) is obtained by the catalytic reduction of an optically active threo-N-carbobenzoxy-3-(3,4-methylenedioxyphenyl)serine (IVb). A catalyst such as palladium, platinum, or nickel, either unsupported or supported on a carrier such as barium carbonate or activated carbon is used in a weight ratio of 0.01—0.1 against unit weight of optically active threo-N-carbobenzoxy-3-(3,4-methylenedioxyphenyl)serine (IVb). Solvents suitable for use in the above reaction include alcohols such as methanol and ethanol; aromatic hydrocarbons such as benzene and toluene; esters such as ethyl acetate; ketones such as acetone and methyl ethyl ketone; water and mixtures of any of these solvents. The reaction proceeds at room temperature or at a temperature higher or lower than room temperature with heating or cooling. The hydrogen pressure can be either atmospheric or superatmospheric. It is possible to add an acid such as hydrochloric acid or sulfuric acid to the reaction system to promote the reaction.

In the next step, the optically active threo-3-(3,4-methylenedioxyphenyl)serine (IIIb) is treated with a Lewis acid as in the aforementioned step D, G, or H to yield an optically active threo-3-(3,4-dihydroxyphenyl)serine (VIb). Examples of suitable Lewis acids are aluminum chloride, aluminum bromide, ferric chloride, stannic chloride, boron trichloride, and boron tribromide. A complex of a Lewis acid and dimethyl sulfide can be used as a Lewis acid. The amount to be used of a Lewis acid is 1 to 20, preferably 2 to 10, moles per 1 mole of threo-3-(3,4-methylenedioxyphenyl)serine (IIIb). In some cases, favorable results are obtained by adding to the reactant mixture, in addition to the Lewis acid, a mercaptan such as methylmercaptan, ethylmercaptan, butylmercaptan, octylmercaptan, dodecanylmercaptan, octadecanylmercaptan or thiophenol in an amount of 1 to 4 moles per 1 mole of the Lewis acid. Although any of the solvents which do not interfere with the progress of reaction may be used as the reaction medium, preferable solvents are halogenated hydrocarbon-type solvents such as dichloromethane, chloroform, dichloroethane, and chlorobenzene; aromatic hydrocarbons such as toluene and benzene; esters such as ethyl acetate and butyl acetate; nitrohydrocarbons such as nitromethane, nitroethane, and nitrobenzene; and ketones such as acetone and methyl ethyl ketone.

Step L (Compound Va → VIa):

Racemic threo-3-(3,4-dihydroxyphenyl)serine (VIa) is prepared from racemic threo-N-carbobenzoxy-3-(3,4-dihydroxyphenyl)serine (Va) by eliminating the carbobenzoxy group under normal conditions such as those described above in Step J or below in Step M.

Step M (Compound Vb → VIb):

An optically active threo-3-(3,4-dihydroxyphenyl)serine (VIb) is prepared by the catalytic reduction of optically active threo-N-carbobenzoxy-3-(3,4-dihydroxyphenyl)serine (Vb). A catalyst such as palladium, platinum, or nickel, either unsupported or supported on a carrier such as barium carbonate or activated carbon is used in a weight ratio of 0.01—0.1 against unit weight of optically active threo-N-carbobenzoxy-3-(3,4-dihydroxyphenyl)serine (Vb). Solvents suitable for use in the above reaction include alcohols such as methanol and ethanol; aromatic hydrocarbons such as benzene and toluene; esters such as ethyl acetate; ketones such as acetone and methyl ethyl ketone; water, and mixtures of any of these solvents. The reaction proceeds at room temperature or at a temperature higher or lower than room temperature with heating or cooling. The hydrogen pressure can be either atmospheric or superatmospheric. It is possible to add an acid such as hydrochloric acid or sulfuric acid to the reaction system to promote the reaction.

Step N (Compound VIa → VIb):

The outline of the procedure for the resolution of racemic threo-3-(3,4-dihydroxyphenyl)serine (VIa) (racemic DOPS) into optically active threo-3-(3,4-dihydroxyphenyl)serine (VIb) (optically active DOPS) is as described previously. The procedure is described below in detail.

The supersaturated solution of racemic DOPS is prepared by the procedures commonly used in preparing a supersaturated solution, such as cooling of a heated solution containing racemic DOPS dissolved therein, or concentration of the solution, or addition of a solvent capable of reducing the solubility of racemic DOPS. Although water is preferred as the solvent, it is possible to add to water those solvents which are uniformly miscible with water. Such solvents include alcohols such as methanol and ethanol; ketones such as acetone, and ethers such as dioxane and tetrahydrofuran. Before use, the solvents should preferably be deaerated. When water is selected as the solvent, it is used in an amount of 50 to 70 times the weight of racemic DOPS.

When the solute of a supersaturated solution of racemic DOPS is optically inactive, it is necessary to add seed crystals to the solution to effect resolution. To the contrary, if either of the optically active isomers exists in major amount, then spontaneous crystallization of the major isomer will take place, whereby the same effect as that of the seeding is obtained without external supply of seed crystals. The term "seeding", as herein used, includes not only the external supply of seed crystals, but also the spontaneous

crystallization. Although with the increase in seeded quantity of crystals, the resolution becomes easier and promoted, yet it is usually sufficient to seed 1 to 10% by weight of racemic DOPS into the solution to be resolved. If the intended optical isomer is present in excess of its antipode, the quantity of seeding to be used prior to the occurrence of spontaneous crystallization can be smaller. The seed crystals of the optical isomer should preferably be of high quality in view of the purpose of seeding.

The present procedure of optical resolution is adaptable to not only the so-called batch-system resolution where either one of the optical isomers is crystallized out of the supersaturated solution throughout the operation, but also simultaneous collection of both optically active DOPS isomers, which is carried out by using two resolving towers or two sections of a tank, both of which are connected in series or parallel, seeding one of the towers or sections with one of the antipodes of optically active DOPS and the other tower or section with the other antipode, and contacting each with a supersaturated racemic DOPS solution.

In carrying out the present procedure, if necessary, the supersaturated racemic DOPS solution may contain an organic base such as dimethylaniline, dimethylbenzylamine, or triethylamine; an inorganic base such as sodium hydroxide or potassium hydroxide; an inorganic acid such as hydrochloric acid or sulfuric acid; an organic acid such as toluenesulfonic acid or methanesulfonic acid; or an amino acid such as D-, L-, or DL-aspartic acid or -glutamic acid in an amount of 0.05—0.6 mole per 1 mole of racemic DOPS, thereby to suppress the crystallization of undesired isomer and, hence, to increase the stability of supersaturated solution. It is also possible to decrease the amount of solvent by the addition of the above-noted organic or inorganic base or acid in an amount of 0.5—1.0 mole per 1 mole of racemic DOPS.

The invention is illustrated below in detail with reference to Examples, but the invention is not limited thereto.

Example 1

Synthesis of racemic erythro- and threo-3-(3,4-methylenedioxyphenyl)serine

To a solution of 61.1 g of potassium hydroxide and 33.05 g of glycine in 1,055 ml of methanol, was added 145 g of piperonal at 30°C or below. The mixture was then stirred at 62° to 65°C for 30 minutes. The reaction mixture was concentrated and the residue was dissolved in 135 ml of methanol. To the solution was added 808 g of acetic acid at 30°C or below. The mixture was then stirred at 40° to 45°C for 30 minutes. After addition of 135 g of water and 1,250 ml of toluene, the mixture was stirred at 40° to 45°C for 2 hours, then at 0° to 5°C for 1 hour. The precipitated crystals were collected by filtration to obtain 86.98 g (69.3% yield) of racemic erythro/threo-3-(3,4-methylenedioxyphenyl)serine acetate.

Mp. 162°C (decomp.)

IR(Nujol) ν ($cm^{-1}$): 3550—2200 (broad), 3490, 1720, 1670, 1580, 1490, 1400, 1290, 1050, 940

HPLC analysis: erythro/threo = 16/84

Conditions for HPLC:

Column: Lichromosorb BP—18, 10 μ, 4 cm × 30 cm

Moving phase solvent: 0.005 M PIC B—7·acetonitrile (9:1)

Flow rate: 1.0 ml/min.

Detection: UV (254 nm)

Elution time: erythro isomer: 7.5 min.
threo isomer: 8.5 min.

The filtrate was left standing at room temperature for 15 hours and the precipitated crystals were collected by filtration to obtain 2.36 g (4.8% yield) of racemic erythro-3-(3,4-methylenedioxyphenyl)serine acetate.

Mp. 138.5°C (decomp.)

IR(Nujol) ν ($cm^{-1}$): 3490, 3400—2200 (broad), 1695, 1590, 1510, 1400, 1240, 1040, 935

NMR(DMSO-$d_6$) δ: 1.88 (3H, s), 3.44 (1H, d, J = 7Hz), 4.89 (1H, d, J = 7Hz), 5.40 (2H, s), 6.74—7.00 (3H, m)

Example 2

(1) A mixture of 1.105 g of water and 85 g of threo/erythro-3-(3,4-methylenedioxyphenyl)serine acetate was heated with stirring under reflux to form a solution. The solution was cooled and stirred at 0° to 5°C for 1 hour. The precipitated crystals were collected by filtration and dried to yield 48.37 g (72.1% yield) of threo-3-(3,4-methylenedioxyphenyl)serine.

Mp. 193°C (decomp.)

IR(Nujol) ν ($cm^{-1}$): 3600—2100 (broad), 3550, 3460, 1630, 1510, 1440, 1400, 1370, 1250, 1040, 930

NMR(DMSO-$d_6$) δ: 3.34 (1H, d, J = 4Hz), 4.97 (1H, d, J = 4Hz), 5.68 (2H, s)

HPLC analysis: erythro/threo = 0/100

(2) A mixture of 20 g of water and 1 g of threo/erythro-3-(3,4-methylenedioxyphenyl)serine acetate was stirred at room temperature for 30 minutes. The precipitated crystals were collected by filtration and dried to give 0.45 g (57.0% yield) of threo-3-(3,4-methylenedioxyphenyl)serine.

Mp. 188°C (decomp.)

HPLC analysis under the conditions given above:

threo:erythro = 98.7:1.3

## Example 3

Synthesis of racemic threo-3-(3,4-methylenedioxyphenyl)serine

A solution of 45.4 g of glycine, 203.2 g of piperonal and 87.7 g of potassium hydroxide in 338 g of methanol was stirred at room temperature for 5 hours. After addition of 637 g of acetic acid, the mixture was stirred at 35° to 45°C for 30 minutes. The reaction mixture was diluted with 181 g of water and stirred at room temperature for 4 hours. Then, 157 g of toluene was added to the mixture and stirring was continued for 2 hours. The precipitated crystals were collected by filtration and washed with toluene to yield 120.6 g of racemic threo-3-(3,4-methylenedioxyphenyl)serine acetate melting at 161°C (decomp.).

IR(Nujol) ν ($cm^{-1}$): 3460—2200 (broad), 1705, 1665, 1610, 1490, 1400, 1285, 1240, 1040, 930, 840

NMR(DMSO-$d_6$) δ: 1.9 (3H, s), 3.47 (1H, d, J = 4Hz), 4.99 (1H, d, J = 4Hz), 5.96 (2H, s), 6.76—6.97 (3H, m)

The filtrate and washings were combined, diluted with 500 g of water, and allowed to separate into two layers. The toluene layer was washed with a 5% aqueous sodium hydroxide solution, then with water, and concentrated under reduced pressure to recover 87.2 g of piperonal.

## Example 4

Synthesis of racemic threo-3-(3,4-dihydroxyphenyl)serine from racemic threo-3-(3,4-methylenedioxyphenyl)serine

To a mixture of 1.5 g of racemic threo-3-(3,4-methylenedioxyphenyl)serine, 1.5 ml of dichloromethane, and 1.5 ml of ethylmercaptan, while being cooled in ice and stirred, was added 7.11 g of anhydrous aluminum bromide. The mixture was stirred with cooling in ice for 3 hours, then at room temperature for 15 hours. After addition of 60 ml of 10% hydrochloric acid, the mixture was allowed to separate into two layers. The aqueous layer was adjusted to pH about 5 with sodium hydroxide. The precipitated crystals were collected by filtration and recrystallized from 30 ml of water containing 15 mg of L-ascorbic acid to yield 1.1 g of racemic threo-3-(3,4-dihydroxyphenyl)serine melting at 226°C (decomp.).

## Example 5

Synthesis of racemic threo-N-carbobenzoxy-3-(3,4-methylenedioxyphenyl)serine

(1) Into a solution of 30.4 g of sodium hydroxide in 1,217.8 g of water, was dissolved 108.6 g of racemic threo-3-(3,4-methylenedioxyphenyl)serine acetate. To the solution cooled in ice, was added dropwise 102 g of a 70% solution of carbobenzoxy chloride in toluene, accompanied by simultaneous dropwise addition of a 30% aqueous sodium hydroxide solution to adjust pH to about 8.5—9.5. After completion of the addition of the carbobenzoxy chloride, the mixture was allowed to react for 4 hours, while the cooling in ice and the pH adjustment being continued. The reaction mixture was then adjusted to pH about 1 with 120 g of concentrated hydrochloric acid, diluted with 350 g of toluene, and stirred at 40—45°C for 1 hour, then at 5—10°C for two hours. The precipitated crystals were collected by filtration, washed with water, then with toluene to yield 347.4 g of racemic threo-N-carbobenzoxy-3-(3,4-methylenedioxyphenyl)serine.

Mp. 136—138°C

IR(Nujol) ν ($cm^{-1}$): 3500, 3250, 1750, 1655, 1505, 1340, 1255, 1220, 1160.

(2) To 160 ml of an aqueous solution containing 4.0 g of sodium hydroxide, while being cooled at 5°C or below, was added 11.3 g of racemic threo-3-(3,4-methylenedioxyphenyl)serine. After complete dissolution, to the solution, while being cooled at 5°C or below, was added dropwise 9.4 g of carbobenzoxy chloride, accompanied by simultaneous dropwise addition of a 30% aqueous sodium hydroxide solution to adjust pH to 8.5—9.5. After 2 hours, the reaction mixture was adjusted to pH about 2 with concentrated hydrochloric acid and extracted with ethyl acetate. The organic layer was washed with saturated aqueous sodium chloride solution, dried over magnesium sulfate, and concentrated under reduced pressure. The residue was crystallized by toluene and the crystals were collected by filtration to give 17.3 g of racemic threo-N-carbobenzoxy-3-(3,4-methylenedioxyphenyl)serine.

Mp.: 136—138°C

IR(Nujol) ν ($cm^{-1}$): 3500, 3250, 1750, 1655, 1505, 1340, 1255, 1220, 1160.

## Example 6

Synthesis of optically active threo-N-carbobenzoxy-3-(3,4-methylenedioxyphenyl)serine

(1) To a solution of 10.0 g of racemic threo-N-carbobenzoxy-3-(3,4-methylenedioxyphenyl)serine in 100 ml of acetonitrile, was added 9.02 g of quinidine to form a uniform solution. The solution was cooled in iced water for 5 hours and the precipitated crystals were collected by filtration to yield 9.0 g of L-threo-N-carbobenzoxy-3-(3,4-methylenedioxyphenyl)serine quinidine salt.

Mp.: 161—163°C

$[\alpha]_D^{20}$ +119.5° (c = 1.0, methanol)

A 2.0 g portion of the above salt was mixed with 5 ml of 3-% hydrochloric acid and extracted with ethyl acetate to obtain 0.98 g of an amorphous powder of L-threo-N-carbobenzoxy-3-(3,4-methylenedioxyphenyl)serine.

IR(Nujol) ν ($cm^{-1}$): 3500—3250 (broad), 1740—1670 (broad), 1390, 1440, 1040, 930.

$[\alpha]_D^{20}$ −24.5° (c = 1.0, methanol)

Another portion, weighing 6.0 g, of the above salt was recrystallized twice from methanol to yield 4.4 g of purified salt; mp. 162—163.5°C, $[\alpha]_D^{20}$ +122.6° (c = 1.0, methanol). A 4.0 g portion of the purified salt was mixed with 3-% hydrochloric acid, and extracted with ethyl acetate to obtain 1.8 g of an amorphous powder of L-threo-N-carbobenzoxy-3-(3,4-methylenedioxyphenyl)serine.

$[\alpha]_D^{20}$ −28.1° (c = 1.0, methanol).

(2) To 10.0 g of racemic threo-N-carbobenzoxy-3-(3,4-methylenedioxyphenyl)serine, were added 100 ml of isopropyl alcohol and 9.02 g of quinidine to form a uniform solution. The solution was left standing overnight at room temperature and the precipitated crystals were collected by filtration to yield 8.3 g of L-threo-N-carbobenzoxy-3-(3,4-methylenedioxyphenyl)serine quinidine salt.

Mp.: 161—163°C

$[\alpha]_D^{20}$ + 19.8° (c = 1.0, methanol).

A 4.0 g portion of the salt was mixed with 3-% hydrochloric acid and extracted with ethyl acetate to obtain 1.9 g of an amorphous powder of L-threo-N-carbobenzoxy-3-(3,4-methylenedioxyphenyl)serine.

$[\alpha]_D^{20}$ −25.3° (c = 1.0, methanol).

(3) To a solution of 5.0 g of racemic threo-N-carbobenzoxy-3-(3,4-methylenedioxyphenyl)serine in 50 ml of acetonitrile, was added 4.5 g of quinine to form a uniform solution. The solution was left standing overnight at room temperature and the precipitated crystals were collected by filtration to yield 5.1 g of D-threo-N-carbobenzoxy-3-(3,4-methylenedioxyphenyl)serine quinine salt.

Mp. 116—118°C

$[\alpha]_D^{20}$ −97.3° (c = 1.0, methanol).

A 4.0 g portion of the salt was mixed with 3-% hydrochloric acid, extracted with ethyl acetate to obtain 2.0 g of an amorphous powder of D-threo-N-carbobenzoxy-3-(3,4-methylenedioxyphenyl)serine.

$[\alpha]_D^{20}$ +18.0° (c = 1.0, methanol).

(4) Into 100 ml of ethanol, were dissolved 10.0 g of racemic threo-N-carbobenzoxy-3-(3,4-methylenedioxyphenyl)serine and 6.3 g of R-2-amino-1,1-diphenylpropanol. The solution was cooled in ice for 4 hours. The precipitated crystals were collected to yield 7.8 g of R-2-amino-1,1-diphenylpropanol salt of L-threo-N-carbobenzoxy-3-(3,4-methylenedioxyphenyl)serine.

M.p. 170.5°C (decomp.)

$[\alpha]_D^{20}$ −23.1° (c = 1.0, methanol).

A 4.0 g portion of the salt was mixed with 100 ml of 3-% hydrochloric acid and extracted with ethyl acetate to yield 2.3 g of L-threo-N-carbobenzoxy-3-(3,4-methylenedioxyphenyl)serine in the form of amorphous powder.

$[\alpha]_D^{20}$ −26.8° (c = 1.0, methanol)

(5) To 10.0 g of racemic threo-N-carbobenzoxy 3-(3,4-methylenedioxyphenyl)serine, were added 4.6 g of l-ephedrine and 200 ml of ethanol. The resulting solution was cooled in ice for 6 hours. The precipitated salt was collected by filtration and recrystallized twice from ethanol to yield 5.3 g of l-ephedrine salt of D-threo-N-carbobenzoxy-3-(3,4-methylenedioxyphenyl)serine.

Mp. 177—178°C

$[\alpha]_D^{20}$ −22.2° (c = 1.0, methanol).

A portion, 2.0 g in weight, of the salt was mixed with 3-% hydrochloric acid and extracted with ethyl acetate to obtain 1.3 g of an amorphous powder of D-threo-N-carbobenzoxy-3-(3,4-methylenedioxophenyl)serine.

$[\alpha]_D^{20}$ +28.0° (c = 1.0, methanol).

Example 7

Synthesis of racemic threo-N-carbobenzoxy-3-(3,4-dihydroxyphenyl)serine

(1) To 18 g of racemic threo-N-carbobenzoxy-3-(3,4-methylenedioxyphenyl)serine, were added 500 ml of dichloromethane and 30 ml of ethylmercaptan. The mixture was stirred while being cooled at 5°C. The resulting solution was mixed with 27.6 g of aluminum chloride and allowed to react at 5°C or below for 4 hours. The reaction mixture was mixed with 250 ml of 10-% hydrochloric acid and allowed to separate into 2 layers. The dichloromethane layer was removed and the aqueous layer was extracted with ethyl acetate. The ethyl acetate layer was washed with saturated aqueous sodium chloride solution, dried over anhydrous magnesium sulfate, and concentrated under reduced pressure, leaving behind 14.7 g of a residue. The residue was purified by silica gel chromatography (elution solvent: acetonitrile/benzene/acetic acid = 4.5/4.5/1) to yield 12.8 g of an amorphous powder of racemic threo-N-carbobenzoxy-3-(3,4-dihydroxyphenyl)serine. The amorphous powder was crystallized in ethyl acetate to give 10.6 g of crystals.

Mp. 145—146°C (decomp.)

IR(Nujol) ν ($cm^{-1}$): 3520, 3490, 3330, 3270, 1720, 1700, 1610, 1535, 1290, 1175, 1080, 1055, 990.

(2) Using 18 g of racemic threo-N-carbobenzoxy-3-(3,4-methylenedioxyphenyl)serine, 500 ml of dichloromethane, 118 g of n-octadecylmercaptan, and 27.6 g of aluminum chloride, the reaction and the

after-treatment were carried out as in (1) above. There was obtained 19.5 g of the residue which was crystallized from ethyl acetate to give 11.7 g of racemic threo-N-carbobenzoxy-3-(3,4-dihydroxyphenyl)-serine; Mp. 145—146°C (decomp.)

(3) The reaction, after-treatment and crystallization were carried out as in (2) above, using 500 ml toluene in place of 500 ml of the dichloromethane used in (2) above. There were obtained 9.0 g of racemic threo-N-carbobenzoxy-3-(3,4-dihydroxyphenyl)serine; Mp. 144—146°C (decomp.).

Example 8

Synthesis of optically active threo-N-carbobenzoxy-3-(3,4-dihydroxyphenyl)serine from optically active threo-N-carbobenzoxy-3-(3,4-methylenedioxyphenyl)serine

(1) To 8.0 g of L-threo-N-carbobenzoxy-3-(3,4-methylenedioxyphenyl)serine [$[\alpha]_D^{20}$ −27.9° (c = 1.0, methanol)], were added 250 ml of dichloromethane and 14 ml of ethylmercaptan. To the mixture, while being cooled at 5°C and stirred, was added 13.0 g of aluminum chloride. After 4 hours of reaction, the reaction mixture was treated and purified as in (1) above to yield 5.1 g of an amorphous powder of L-threo-N-carbobenzoxy-3-(3,4-dihydroxyphenyl)serine.

IR(Nujol) ν ($cm^{-1}$): 3500—3250 (broad), 1740—1670 (broad), 1390, 1440, 1040, 930

$[\alpha]_D^{20}$ −27.4° (c = 1.0, methanol)

(2) To 1.0 g of D-threo-N-carbobenzoxy-3-(3,4-methylenedioxyphenyl)serine [$[\alpha]_D^{20}$ +28.0° (c = 1.0, methanol)], were added 25 ml of methylene chloride and 3.4 g of n-octylmercaptan. To the mixture, while being cooled at 5°C and stirred, was added 1.5 g of aluminum chloride. After 6 hours of stirring, the reaction mixture was treated and purified as in (1) above to obtain 0.56 g of an amorphous powder of D-threo-N-carbobenzoxy-3-(3,4-dihydroxyphenyl)serine.

IR(Nujol) ν ($cm^{-1}$): 3500—3250 (broad), 1740—1670 (broad), 1390, 1440, 1040, 930

$[\alpha]_D^{20}$ +27.3° (c = 1.0, methanol).

(3) Using 8.0 g of L-threo-N-carbobenzoxy-3-(3,4-methylenedioxyphenyl)serine [$[\alpha]_D^{20}$ −27.9° (c = 1.0, methanol)], 250 ml of dichloromethane, 36.8 g of n-dodecylmercaptan, and 12.3 g of aluminum chloride, the reaction, after-treatment and purification were carried out as in (1) above to obtain 6.2 g of an amorphous powder of L-threo-N-carbobenzoxy-3-(3,4-dihydroxyphenyl)serine.

$[\alpha]_D^{20}$ −27.5° (c = 1.0, methanol).

(4) To a solution of 10.0 g of L-threo-N-carbobenzoxy-3-(3,4-methylenedioxyphenyl)serine [$[\alpha]_D^{20}$ −27.9° (c = 1.0, methanol)] in 200 ml of methylene chloride, were added at room temperature 31 g of pyridine and 46 g of aluminum chloride. The mixture was stirred for 3 hours and, while being cooled in ice, mixed with 200 ml of 3-% hydrochloric acid. The mixture was allowed to separate into layers and the aqueous layer was extracted with ethyl acetate. The ethyl acetate layer was washed with water, dried over anhydrous magnesium sulfate, and stripped of the solvent by concentration under reduced pressure. The residue was purified by silica gel column chromatography (eluent: acetonitrile/benzene/acetic acid = 4.5/4.5/1) to give 2.8 g of an amorphous powder of L-threo-N-carbobenzoxy-3-(3,4-dihydroxyphenyl)serine.

$[\alpha]_D^{20}$ −27.0° (c = 1.0, methanol).

(5) To 1.0 g of L-threo-N-carbobenzoxy-3-(3,4-methylenedioxyphenyl)serine [$[\alpha]_D^{20}$ −26.8° (c = 1.0, methanol)], were added 50 ml of dichloromethane and 2.7 ml of ethylmercaptan. To the mixture, while being cooled at 5°C or below in iced water, was added 1.5 g of aluminum bromide. After 30 minutes of reaction, the reaction mixture was mixed with 50 ml of 3-% hydrochloric acid. The after-treatment and purification were carried out as in (4) above to yield 0.58 g L-threo-N-carbobenzoxy-3-(3,4-dihydroxyphenyl)serine.

$[\alpha]_D^{20}$ −25.7° (c = 1.0, methanol).

(6) To a solution of 1.0 g of L-threo-N-carbobenzoxy-3-(3,4-methylenedioxyphenyl)serine [$[\alpha]_D^{20}$ −26.8° (c = 1.0, methanol)] in 50 ml of dichloromethane, while being cooled at 5°C or below in iced water, was added 2.2 g of titanium tetrachloride. The mixture was allowed to react at room temperature for 1 hour. The reaction mixture was mixed with 50 ml of 3-% hydrochloric acid, while being cooled in iced water, and allowed to separate into layers. The aqueous layer was extracted with ethyl acetate and the extract was washed with water, dried, and stripped of the solvent by distillation. The residue was purified by silica gel chromatography to yield 0.22 g of L-threo-N-carbobenzoxy-3-(3,4-dihydroxyphenyl)serine in the form of amorphous powder.

$[\alpha]_D^{20}$ −26.3° (c = 1.0, methanol).

Example 9

Synthesis of optically active threo-N-carbobenzoxy-3-(3,4-dihydroxyphenyl)serine from racemate

(1) Into a mixture of 400 ml of methanol and 250 ml of water, was dissolved at room temperature 50.0 g of racemic threo-N-carbobenzoxy-3-(3,4-dihydroxyphenyl)serine followed by the addition of 41.8 g of

cinchonidine. After 20 minutes of stirring, the mixture was heated to 50°C and allowed to cool gradually down to 20°C over a period of 2 hours. The mixture was stirred at 15° to 20°C for 3 hours. The precipitated crystals were collected by filtration to yield 40.2 g of L-threo-N-carbobenzoxy-3-(3,4-dihydroxyphenyl)-serine cinchonidine salt; mp. 140°C (decomp.); $[\alpha]_D^{20}$ −64.0° (c = 1.0, methanol).

A 22 g portion of the cinchonidine salt was decomposed by the addition of 80 ml of 3-% hydrochloric acid and extracted with 240 ml of ethyl acetate. The extract gave 11.3 g of amorphous powder of L-threo-N-carbobenzoxy-3-(3,4-dihydroxyphenyl)serine; $[\alpha]_D^{20}$ −25.9° (c = 1.0, methanol).

IR(Nujol) ν ($cm^{-1}$): 3600—3100 (broad), 1600—1760 (broad), 1600, 1520, 1340, 1270, 1050.

The mother liquid from which the crystallized cinchonidine salt had been removed by filtration was stripped of the methanol by distillation under reduced pressure, admixed with 120 ml of 3-% hydrochloric acid, and extracted with ethyl acetate to yield 29.3 g of an amorphous powder of D-threo-N-carbobenzoxy-3-(3,4-dihydroxyphenyl)serin.

$[\alpha]_D^{20}$ +17.4° (c = 1.0, methanol)

IR(Nujol) ν ($cm^{61}$): 3600—3100 (broad), 1760—1600 (broad), 1600, 1520, 1340, 1270, 1050.

To a solution of 8.9 g of the above D-threo-N-carbobenzoxy-3-(3,4-dihydroxyphenyl)serine in 260 ml of ethyl acetate, was added at room temperature 2.7 g of benzylamine. The precipitated crystals were collected by filtration to yield 10.9 g of D-threo-N-carbobenzoxy-3-(3,4-dihydroxyphenyl)serine benzylamine salt; mp. 145°C (decomp.); $[\alpha]_D^{20}$ −5.3° (c = 1.0, methanol).

(2) To a solution of 0.5 g of racemic threo-N-carbobenzoxy-3-(3,4-dihydroxyphenyl)serine in 5 ml of dioxane, was added 0.62 g of brucine. The mixture was stirred at room temperature for 15 hours. The precipitated crystals were collected by filtration to yield 0.21 g of D-threo-N-carbobenzoxy-3-(3,4-dihydroxyphenyl)serine brucine salt; mp. 139°C (decomp.); $[\alpha]_D^{20}$ −1.9° (c = 1.0, methanol).

A 2.0 g portion of the salt was decomposed with 3-% hydrochloric acid, and extracted with ethyl acetate to recover 0.08 g of an amorphous powder of D-threo-N-carbobenzoxy-3-(3,4-dihydroxyphenyl)-serine; $[\alpha]_D^{20}$ +25.5° (c = 1.0, methanol).

(3) To a solution of 0.5 g of racemic threo-N-carbobenzoxy-3-(3,4-dihydroxyphenyl)serine in 5 ml of isopropyl alcohol, was added 0.33 g of S-2-amino-1,1-diphenylpropanol. The mixture was stirred at room temperature for 1 hour. The precipitated crystals were collected by filtration to yield 0.30 g of L-threo-N-carbobenzoxy-3-(3,4-dihydroxyphenyl)serine S-amino-1,1-diphenylpropanol salt; mp. 167°C (decomp.); $[\alpha]_D^{20}$ +31.5° (c = 1.0, methanol).

A 0.2 g portion of the salt was decomposed with 3-% hydrochloric acid and extracted with ethyl alcohol to yield 0.12 g of amorphous powder of L-threo-N-carbobenzoxy-3-(3,4-dihydroxyphenyl)serine; $[\alpha]_D^{20}$ −26.3° (c = 1.0, methanol).

(4) To a solution of 0.5 g of racemic threo-N-carbobenzoxy-3-(3,4-dihydroxyphenyl)serine in 10 ml of acetonitrile, was added 0.48 g of l-ephedrine. The mixture was left standing at room temperature for 10 days. The precipitated crystals were collected by filtration to yield 0.24 g of D-threo-N-carbobenzoxy-3-(3,4-dihydroxyphenyl)serine l-ephedrine salt; mp. 104°C (decomp.); $[\alpha]_D^{20}$ −21.5° (c = 1.0, methanol).

A portion of the salt was decomposed with 3-% hydrochloric acid and extracted with ethyl acetate to yield 0.1 g of D-threo-N-carbobenzoxy-3-(3,4-dihydroxyphenyl)serine; $[\alpha]_D^{20}$ +20.5° (c = 1.0, methanol).

Example 10

Preparation of optically active threo-3-(3,4-methylenedioxyphenyl)serine from optically active threo-N-carbobenzoxy-3-(3,4-methylenedioxyphenyl)serine

To a solution of 12.7 g of L-threo-3-(3,4-methylenedioxyphenyl)-N-carbobenzoxyserine [$[\alpha]_D^{20}$ −28.1° (c = 1.0, methanol)] in 110 ml of methanol, were added 1.27 g of 10% Pd-C (50% wet) and 12.7 g of water. The mixture was subjected to hydrogenolysis at atmospheric pressure. After the hydrogen had been absorbed no more, 5.03 g of concentrated hydrochloric acid was added to the reaction mixture, stirred, and removed of the insolubles by filtration. The filtrate was adjusted to pH about 5.5 with a 30% aqueous sodium hydroxide solution. The precipitated crystals were collected by filtration and recrystallized from water to yield 5.20 g of L-threo-3-(3,4-methylenedioxyphenyl)serine; mp. 196—198°C (decomp.); $[\alpha]_D^{25}$ −31.3° (c = 1.0, N hydrochloric acid).

Example 11

Preparation of optically active threo-3-(3,4-dihydroxyphenyl)serine from optically active threo-3-(3,4-methylenedioxyphenyl)serine

To a solution of 4.50 g of the L-threo-3-(3,4-methylenedioxyphenyl)serine in 25 ml of 1,2-dichloroethane, while being cooled at −15°C, was added dropwise a solution of 12.5 g of boron trichloride in 1,2-dichloroethane. The mixture was stirred at −15° to −10° for 30 minutes, diluted with 40 ml of saturated aqueous sodium chloride solution, and further stirred at 0° to 5°C for 20 minutes. The precipitate was collected by filtration, then dissolved in 50 ml of water, and, while being cooled at 0° to 5°C, adjusted to pH about 2 with a 30% aqueous sodium hydroxide solution, and 1.2 g of calcium hydroxide was dissolved in the mixture. The mixture was adjusted to pH about 9.5 with a 30% aqueous sodium hydroxide solution

and left standing overnight at 0° to 4°C. The precipitated crystals were removed by filtration and the filtrate was adjusted to pH 4.7 with concentrated hydrochloric acid and left standing overnight at 0° to 3°C. The precipitated crystals were collected by filtration to yield 1.5 g of crude L-threo-3-(3,4-dihydroxyphenyl)-serine which gave, upon recrystallization from water, 0.30 g of L-threo-3-(3,4-dihydroxyphenyl)serine; mp. 228—233°C; $[\alpha]_D^{25}$ −39.0 (c = 1.0, N hydrochloric acid).

Example 12

Preparation of racemic threo-3-(3,4-dihydroxyphenyl)serine from racemic threo-N-carbobenzoxy-3-(3,4-dihydroxyphenyl)serine.

To a mixture of 11.8 g of racemic threo-N-carbobenzoxy-3-(3,4-dihydroxyphenyl)serine [mp. 145—146°C (decomp.)], 120 ml of methanol, and 12.0 ml of water, was added 1.2 g of 5-% palladium-carbon (50% wet). The mixture was subjected to catalytic reduction under a hydrogen stream. After completion of the reaction, the reaction mixture was mixed with 4.0 g of concentrated hydrochloric acid and stirred for 20 minutes. The insolubles were removed by filtration and washed with methanol. The filtrate and the washings were combined, adjusted to pH 5.5—6.0 with a 30% aqueous sodium hydroxide solution, and stirred at 0° to 5°C for 2 hours. The precipitated crystals were collected by filtration to give 6.8 g of racemic threo-3-(3,4-dihydroxyphenyl)serine [mp. 211—220°C (decomp.)].

Example 13

Synthesis of optically active threo-3-(3,4-dihydroxyphenyl)serine

(1) To a mixture of 2.36 g of L-threo-N-carbobenzoxy-3-(3,4-dihydroxyphenyl)serine [$[\alpha]_D^{20}$ −27.4° (c = 1.0, methanol)], 24 ml of methanol, and 2.4 ml of water, was added 0.24 g of 5% palladium-carbon (50% wet). The mixture was subjected to catalytic reduction under a hydrogen stream. After completion of the reaction, the reaction mixture was mixed with 0.8 g of concentrated hydrochloric acid and stirred for 20 minutes. The insolubles were separated by filtration and washed with methanol. The filtrate and the washings were combined and adjusted to pH 5.5—6.0 with a 30-% aqueous sodium hydroxide solution to precipitate crystals. The mixture was stirred at 0° to 5°C for 2 hours. The precipitated crystals were collected by filtration to give 1.33 g (91.9% yield) of L-threo-3-(3,4-dihydroxyphenyl)serine; $[\alpha]_D^{20}$ −39.7° (c = 1.0, 1N hydrochloric acid); mp. 215° (decomp.).

A 1.2 g portion of the L-threo-3-(3,4-dihydroxyphenyl)serine was recrystallized from 76 ml of water containing 0.01 g of L-ascorbic acid to give 0.96 g (80.0% yield) of purified L-threo-3-(3,4-dihydroxyphenyl-serine [$[\alpha]_D^{20}$ −40.7° (c = 1.0, *N* hydrochloric acid); mp. 226°C (decomp.)].

(2) To a mixture of 3.0 g of L-threo-N-carbobenzoxy-3-(3,4-dihydroxyphenyl)serine [$[\alpha]_D^{20}$ −27.5° (c = 1.0, methanol)], 15 ml of methanol, 15 ml of ethyl acetate, and 3 ml of water, was added 0.3 g of 5% palladium-carbon (50% wet). The mixture was subjected to catalytic reduction under a hydrogen stream. After completion of the reaction, the reaction mixture was mixed with 1.0 g of concentrated hydrochloric acid and stirred for 20 minutes. The insolubles were separated and washed with methanol. The filtrate and the washings were combined and adjusted to pH 5.5—6.0 with a 30% aqueous sodium hydroxide solution to precipitate crystals. After two hours of stirring at 0° to 5°C, the precipitated crystals were collected by filtration to give 1.67 g (90.5% yield) of L-threo-3-(3,4-dihydroxyphenyl)serine [$[\alpha]_D^{20}$ −37.3° (c = 1.0, N hydrochloric acid); mp. 214°C (decomp.)].

A 1.5 g portion of this compound was recrystallized from 96 ml of water containing 0.02 g of L-ascorbic acid to give 1.2 g (80.5% yield) of purified L-threo-3-(3,4-dihydroxyphenyl)serine; $[\alpha]_D^{20}$ −40.4° (c = 1.0, N hydrochloric acid); mp. 226°C (decomp.).

Example 14

(1) A 1.0 g portion of racemic threo-3-(3,4-dihydroxyphenyl)serine (briefly racemic DOPS) was added to 60 ml of deaerated water and heated at 85°C to form a solution. The hot solution was gradually cooled down to 70°C, seeded with 0.10 g of L-DOPS, then further cooled down to 50°C over a period of 45 minutes. The precipitated crystals were collected to yield 0.167 g of L-DOPS.

Mp.: 232—4°C (decomp.)

$[\alpha]_D^{28}$: −33.1° (c = 1.0, N-hydrochloric acid)

Optical purity: 87.0%.

(2) To 100 ml of deaerated water, were added 2.0 g of racemic DOPS and 0.40 g of L-aspartic acid. The mixture was heated at 85°C to form a solution. The hot solution was gradually cooled down to 70°C, seeded with 0.1 g of L-DOPS, and further cooled down to 53°C over a period of 45 minutes. The precipitated crystals were collected by filtration to yield 0.207 g of L-DOPS.

Mp.: 232—4°C (decomp.)

$[\alpha]_D^{28}$: −33.4° (c = 1.0, N-hydrochloric acid)

Optical purity: 87.8%.

To the filtrate separated from the L-DOPS crystals, was added 0.107 g of racemic DOPS. The mixture was heated at 85°C to form a solution. The hot solution was gradually cooled down to 70°C, seeded with

0.10 g of D-DOPS, then further cooled down to 53°C over a period of 45 minutes. The precipitated crystals were collected by filtration to yield 0.297 g of D-DOPS.

Mp.: 232—4°C (decomp.)
$[\alpha]_D^{28}$: +37.9° (c = 1.0, N-hydrochloric acid)
Optical purity: 93.8%.

(3) To 50 ml of deaerated water, were added 1.0 g of racemic DOPS and 0.10 g of L-glutamic acid. The mixture was heated at 85°C to form a solution. The hot solution was gradually cooled down to 70°C, seeded with 0.05 g of L-DOPS, then further cooled down to 60°C over a period of 45 minutes. The precipitated crystals were collected to yield 0.098 g of L-DOPS.

Mp.: 232—4°C (decomp.)
$[\alpha]_D^{28}$: −28.8° (c = 1.0, N hydrochloric acid)
Optical purity: 75.6%.

(4) To 15 ml of deaerated water, were added 1.0 g of racemic DOPS and 0.242 g of concentrated sulfuric acid. The mixture was heated at 85°C to form a solution. The hot solution was gradually cooled down to 30°C, seeded with 0.05 g of L-DOPS, then further cooled down to 25°C over a period of 45 minutes. The precipitated crystals were collected by filtration to yield 0.083 g of L-DPOS.

Mp.: 232—4°C (decomp.)
$[\alpha]_D^{28}$: −28.3° (c = 1.0, N hydrochloric acid)
Optical purity: 74.2%.

(5) To 10 ml of deaerated water, were added 1.0 g of racemic DOPS and 0.89 g of p-toluenesulfonic acid. The mixture was heated at 70°C to form a solution. The hot solution was gradually cooled down to 25°C, seeded with 0.05 g of L-DOPS, and cooled at 0° to 4°C for 18 hours. The precipitated crystals were collected by filtration to yield 0.164 g of L-DOPS.

Mp.: 232—4°C (decomp.)
$[\alpha]_D^{28}$: −25.0° (c = 1.0, N hydrochloric acid)
Optical purity: 65.6%.

**Claims**

1. A process for producing threo-3-(3,4-dihydroxyphenyl)serine represented by the formula

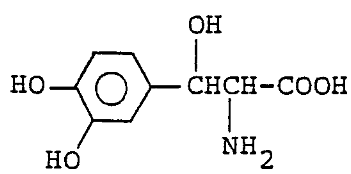

[1],

which comprises treating threo-3-(3,4-methylenedioxyphenyl)serine or an N-carbobenzoxy derivative thereof represented by the formula

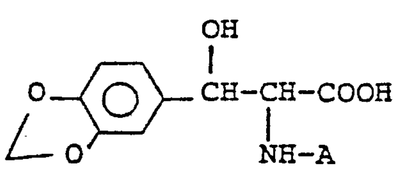

[3],

wherein A represents a hydrogen atom or a carbobenzoxy group, with a Lewis acid to form threo-3-(3,4-dihydroxyphenyl)serine or an N-carbobenzoxy derivative thereof represented by the formula

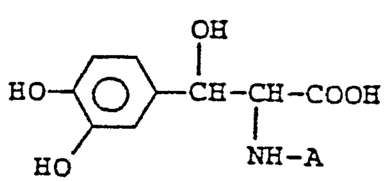

[2],

wherein A is as defined above, and, if A is a carbobenzoxy group, catalytically reducing the resulting compound of formula [2].

2. A process according to Claim 1, wherein at least one of the compounds represented by the formulae [1], [2] and [3] is an optically active compound.

3. A process according to Claim 1, wherein A of the formulae [2] and [3] is a hydrogen atom.

4. A process according to Claim 1, wherein A of the formulae [2] and [3] is a carbobenzoxy group.

5. A process according to Claim 4, wherein at least one of the compounds represented by the formulae [1], [2] and [3] is an optically active compound.

6. A process for producing an optically active threo-3-(3,4-dihydroxyphenyl)serine represented by the formula

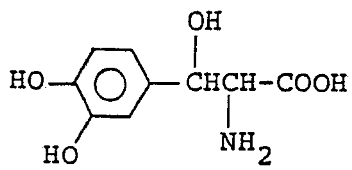

[1],

which comprises reacting racemic threo-N-carbobenzoxy-3-(3,4-methylenedioxyphenyl)serine with one optically active amine selected from ephedrine, quinidine, quinine and 2-amino-1,1-diphenylpropanol to form a mixture of amine salts of D- and L-threo-N-carbobenzoxy-3-(3,4-methylenedioxyphenyl)serine, separating the mixture by taking advantage of the solubility difference into the amine salt of D-threo-N-carbobenzoxy-3-(3,4-methylenedioxyphenyl)serine and the amine salt of L-threo-N-carbobenzoxy-3-(3,4-methylenedioxyphenyl)serine, reacting each amine salt with an acid to obtain an optically active (D- or L-)threo-N-carbobenzoxy-3-(3,4-methylenedioxyphenyl)serine, reacting the resulting optically active compound with a Lewis acid to yield an optically active threo-N-carbobenzoxy-3-(3,4-dihydroxyphenyl)-serine, and catalytically reducing the resulting compound.

7. A process for producing an optically active threo-3-(3,4-dihydroxyphenyl)serine represented by the formula

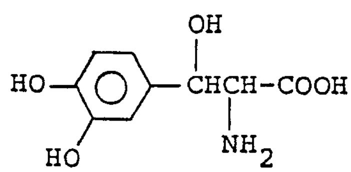

[1],

which comprises treating racemic threo-N-carbobenzoxy-3-(3,4-methylenedioxyphenyl)serine with a Lewis acid to yield racemic threo-N-carbobenzoxy-3-(3,4-dihydroxyphenyl)serine, reacting the resulting compound with one optically active amine selected from cinchonidine, brucine, ephedrine, and 2-amino-1,1-diphenylpropanol to convert the racemic mixture into a mixture of amine salts of D- and L-threo-N-carbobenzoxy-3-(3,4-dihydroxyphenyl)serine, separating the mixture by taking advantage of the solubility difference into the amine salt of D-threo-N-carbobenzoxy-3-(3,4-dihydroxyphenyl)serine and the amine salt of L-threo-N-carbobenzoxy-3-(3,4-dihydroxyphenyl)serine, reacting each amine salt with an acid to obtain an optically active threo-N-carbobenzoxy-3-(3,4-dihydroxyphenyl)serine, and catalytically reducing the resulting optically active compound.

8. A process for producing an optically active threo-3-(3,4-dihydroxyphenyl)serine represented by the formula

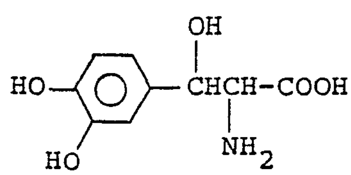

[1],

which comprises reacting glycine with piperonal in the presence of a base, adding acetic acid to the reaction mixture to obtain acetate of the racemic threo-3-(3,4-methylenedioxyphenyl)serine represented by the formula

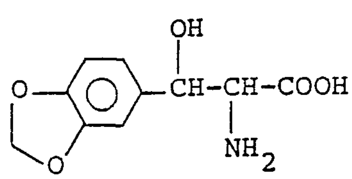

[3'],

reacting said acetate with carbobenzoxy chloride to yield racemic threo-N-carbobenzoxy-3-(3,4-methylenedioxyphenyl)serine represented by the formula

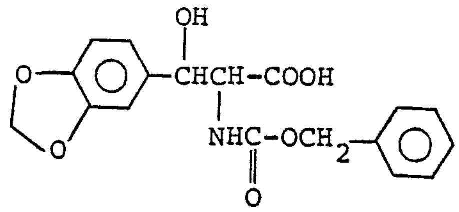

[3"]

subjecting the resulting racemic mixture to optical resolution by use of an optically active amine selected from ephedrine, quinidine, quinine, and 2-amino-1,1-diphenylpropanol to yield an optically active threo-N-carbobenzoxy-3-(3,4-methylenedioxyphenyl)serine, treating the resulting optically active compound with a Lewis acid to convert the former to optically active threo-N-carbobenzoxy-3-(3,4-dihydroxyphenyl)serine represented by the formula

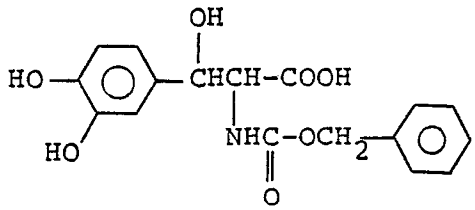

[2']

and catalytically reducing the resulting optically active compound.

9. A process for producing an optically active threo-3-(3,4-dihydroxyphenyl)serine represented by the formula

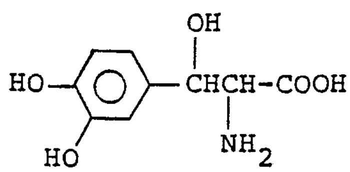

[1],

which comprises reacting glycine with piperonal in the presence of a base, adding acetic acid to the reaction mixture to obtain acetate of the racemic threo-3-(3,4-methylenedioxyphenyl)serine represented by the formula

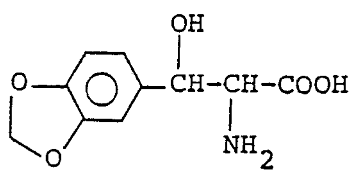

[3'],

reacting said acetate with carbobenzoxy chloride to yield racemic threo-N-carbobenzoxy-3-(3,4-methylenedioxyphenyl)serine represented by the formula

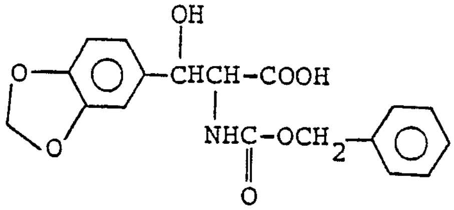

[3"]

treating the resulting compound with a Lewis acid to yield racemic threo-N-carbobenzoxy-3-(3,4-dihydroxyphenyl)serine, subjecting the resulting racemic mixture to optical resolution by use of an optically active amine selected from cinchonidine, brucine, ephedrine, and 2-amino-1,1-diphenylpropanol to obtain an optically active threo-N-carbobenzoxy-3-(3,4-dihydroxyphenyl)serine, and catalytically reducing the resulting optically active compound.

10. A process for producing racemic or optically active threo-3-(3,4-dihydroxyphenyl)serine represented by the formula

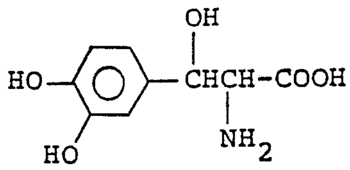

[1],

which comprises reacting glycine with piperonal in the presence of a base, adding acetic acid to the reaction mixture to obtain acetate of the racemic threo-3-(3,4-methylenedioxyphenyl)serine represented by the formula

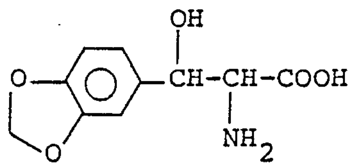

[3'],

and treating said acetate, either as such or after having been resolved into optically active isomers, with a Lewis acid.

11. A process for producing an optically active threo-3-(3,4-dihydroxyphenyl)serine, which comprises allowing the crystals of an optically active threo-3-(3,4-dihydroxyphenyl)serine to be present in a supersaturated solution of racemic threo-3-(3,4-dihydroxyphenyl)serine, and allowing the same optically active threo-3-(3,4-dihydroxyphenyl)serine as said crystals to crystallize preferentially out of said supersaturated solution.

12. Threo-N-carbobenzoxy-3-(3,4-methylenedioxyphenyl)serine represented by the formula

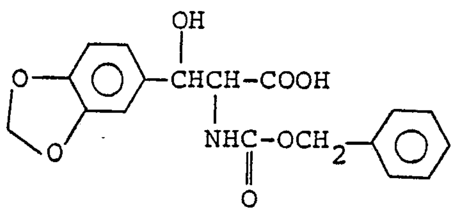

[3"]

13. An optically active threo-N-carbobenzoxy-3-(3,4-methylenedioxyphenyl)serine represented by the formula

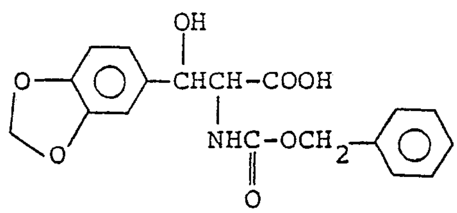

[3"]

14. A process for producing a threo-N-carbobenzoxy-3-(3,4-methylenedioxyphenyl)serine represented by the formula

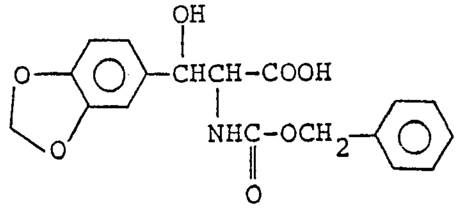

[3"]

which comprises reacting glycine with piperanol in the presence of a base, adding acetic acid to the reaction mixture to obtain acetate of racemic threo-3-(3,4-methylenedioxyphenyl)serine, reacting said acetate with carbobenzoxy chloride, and, if necessary, subjecting the resulting racemic threo-N-carbobenzoxy-3-(3,4-methylenedioxyphenyl)serine to optical resolution through the salt formation with an optically active amine.

**Patentansprüche**

1. Verfahren zur Herstellung von threo-3-(3,4-Dihydroxyphenyl)serin der Formel

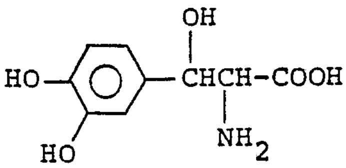

[1],

welches die Behandlung von threo-3-(3,4-Methylendioxyphenyl)serin oder einem N-Carbobenzoxyderivat davon der allgemeinen Formel

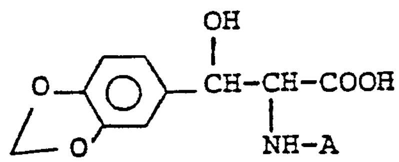

[3],

worin A ein Wasserstoffatom oder eine Carbobenzoxygruppe bedeutet, mit einer Lewissäure zu threo-3-(3,4-Dihydroxyphenyl)serin oder einem N-Carbobenzoxyderivat davon der allgemeinen Formel

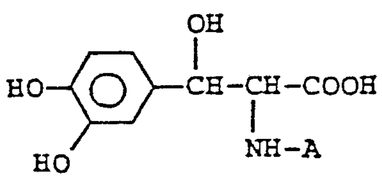

[2],

worin A die oben angegebene Bedeutung hat, und, wenn A eine Carbobenzoxygruppe ist, die katalytische Reduktion der sich ergebenden Verbindung der Formel (2) umfaßt.

2. Verfahren nach Anspruch 1, worin wenigstens eine der durch die Formeln (1), (2) und (3) dargestellten Verbindungen eine optisch aktive Verbindung ist.

3. Verfahren nach Anspruch 1, worin A der Formeln (2) und (3) ein Wasserstoffatom ist.

4. Verfahren nach Anspruch 1, worin A der Formeln (2) und (3) eine Carbobenzoxygruppe ist.

5. Verfahren nach Anspruch 4, worin wenigstens eine der durch die Formeln (1), (2) und (3) dargestellten Verbindungen eine optisch aktive Verbindung ist.

6. Verfahren zur Herstellung eines optisch aktiven threo-3-(3,4-Dihydroxyphenyl)serins der Formel

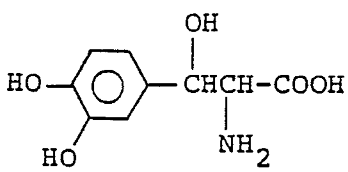

[1],

welches Umsetzen von racemischem threo-N-Carbobenzoxy-3-(3,4-methylendioxyphenyl)serin mit einem optisch aktiven, aus Ephedrin, Chinidin, Chinin und 2-Amino-1,1-diphenylpropanol ausgewählten Amin zu einem Gemisch von Aminsalzen von D- und L-threo-N-Carbobenzoxy-3-(3,4-methylendioxyphenyl)serin, Auftrennen des Gemisches durch Ausnützen der Löslichkeitsunterschiede in Aminsalze von D-threo-N-Carbobenzoxy-3-(3,4-methylendioxyphenyl)serin und das Aminsalz von L-threo-N-Carbobenzoxy-3-(3,4-methylendioxyphenyl)serin, Umsetzen jedes Aminsalzes mit einer Säure zu einem optisch aktiven (D- oder

L-)-threo-N-Carbobenzoxy-3-(3,4-methylendioxyphenyl)serin, Umsetzen der sich ergebenden optisch aktiven Verbindung mit einer Lewissäure zu einem optisch aktiven threo-N-Carbobenzoxy-3-(3,4-dihydroxyphenyl)serin und katalytische Reduktion der sich ergebenden Verbindung umfaßt.

7. Verfahren zur Herstellung eines optisch aktiven threo-3-(3,4-Dihydroxyphenyl)serin der Formel

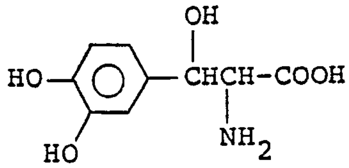

[1],

welches Behandeln des racemischen threo-N-Carbobenzoxy-3-(3,4-methylendioxyphenyl)serin mit einer Lewissäure zum racemischen threo-N-Carbobenzoxy-3-(3,4-dihydroxyphenyl)serin, Umsetzen der sich ergebenden Verbindung mit einem optisch aktiven, aus Cinchonidin, Brucin, Ephedrin und 2-Amino-1,1-diphenylpropanol ausgewählten Amin zur Umsetzung des racemischen Gemisches zu einem Gemisch von Aminsalzen von D- und L-threo-N-Carbobenzoxy-3-(3,4-dihydroxyphenyl)serin, Auftrennen des Gemisches durch Ausnützen der Löslichkeitsunterschiede in das Aminsalz von D-threo-N-Carbobenzoxy-3-(3,4-dihydroxyphenyl)serin und das Aminsalz von L-threo-N-Carbobenzoxy-3-(3,4-dihydroxyphenyl)serin, Umsetzen jedes Aminsalzes mit einer Säure zu einem optisch aktiven threo-N-Carbobenzoxy-3-(3,4-dihydroxyphenyl)serin und katalytische Reduktion der sich ergebenden optisch aktiven Verbindung umfaßt.

8. Verfahren zur Herstellung eines optisch aktiven threo-3-(3,4-Dihydroxyphenyl)serin der Formel

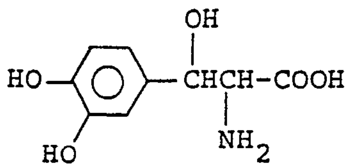

[1],

welches die Umsetzung von Glycin mit Piperonal in Gegenwart einer Base, Zugabe von Essigsäure zum Reaktionsgemisch zur Bildung des Acetates des racemischen threo-3-(3,4-Methylendioxyphenyl)serin der Formel

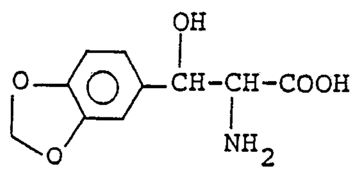

[3'],

Umsetzen des Acetates mit Carbobenzoxychlorid zum racemischen threo-N-Carbobenzoxy-3-(3,4-methylendioxyphenyl)serin der Formel

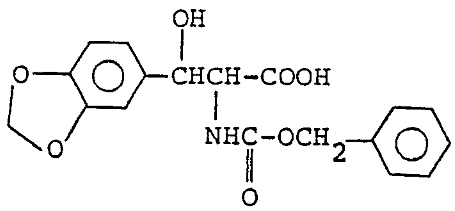

[3"]

Auftrennen des erhaltenen racemischen Gemisches in optische Antipoden durch Verwendung eines optisch aktiven, aus Ephedrin, Chinidin, Chinin und 2-Amino-1,1-diphenylpropanol ausgewählten Amins zu einem optisch aktiven threo-N-Carbobenzoxy-3-(3,4-methylendioxyphenyl)serin, Behandeln der erhaltenen optisch aktiven Verbindung mit einer Lewissäure zur Umsetzung zu einem optisch aktiven threo-N-Carbobenzoxy-3-(3,4-dihydroxyphenyl)serin der Formel threo-3-(3,4-Dihydroxyphenyl)serin der Formel

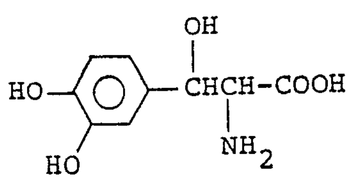

[1],

welches die Umsetzung von Glycin mit Piperonal in Gegenwart einer Base, Zugabe von Essigsäure zum Reaktionsgemisch zur Bildung des Acetates des racemischen threo-3-(3,4-Methylendioxyphenyl)serin der Formel

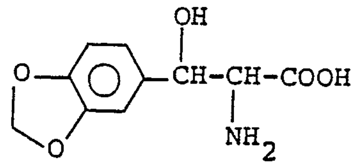

[3'],

und Behandeln des Acetates entweder als solches oder nach Auftrennung in optisch aktive Isomere mit einer Lewissäure umfaßt.

11. Verfahren zur Herstellung eines optisch aktiven threo-3-(3,4-Dihydroxyphenyl)serin, welches Zulassen der Anwesenheit der Kristalle eines optisch aktiven threo-3-(3,4-Dihydroxyphenyl)serin in einer übersättigten Lösung von racemischem threo-3-(3,4-Dihydroxyphenyl)serin und Auskristallisierenlassen des optisch aktiven threo-3-(3,4-Dihydroxyphenyl)serin als besagte Kristalle vorzugsweise aus der übersättigten Lösung umfaßt.

12. Threo-N-Carbobenzoxy-3-(3,4-methylendioxyphenyl)serin der Formel

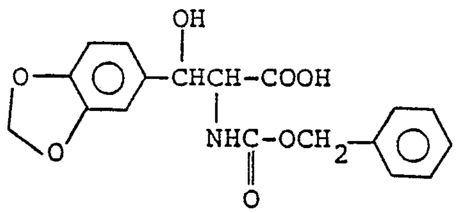

[3"]

13. Optisch aktives threo-N-Carbobenzoxy-3-(3,4-methylendioxyphenyl)serin der Formel

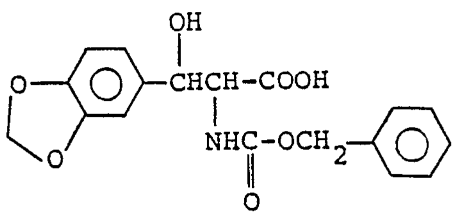

[3"]

14. Verfahren zur Herstellung eines threo-N-Carbobenzoxy-3-(3,4-methylendioxyphenyl)serin der Formel

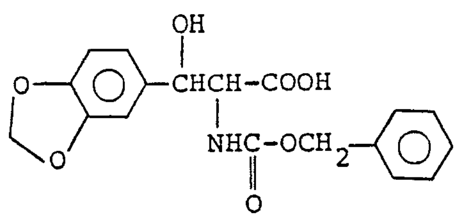

[3"]

welches die Umsetzung von Glycin mit Piperonal in Gegenwart einer Base, Zugabe von Essigsäure zum Reaktionsgemisch zur Bildung des Acetates des racemischen threo-3-(3,4-Methylendioxyphenyl)serin, Umsetzung des Acetates mit Carbobenzoxychlorid und, wenn nötig, Auftrennen des erhaltenen threo-N-Carbobenzoxy-3-(3,4-methylendioxyphenyl)serin in optische Antipoden durch Salzbildung mit einem optisch aktiven Amin umfaßt.

**Revendications**

1. Procédé pour la préparation de thréo-3-(3,4-dihydroxyphényl)-sérine représentée par la formule

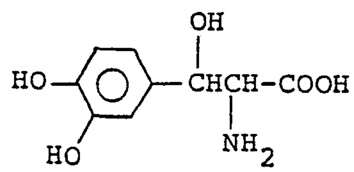

[1],

consistant à traiter la thréo-3-(3,4-méthylènedioxyphényl)-sérine ou un dérivé N-carbobenzoxy de celle-ci représentés par la formule

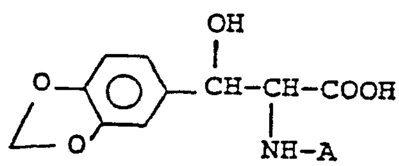

[3],

dans laquelle A représente un atome d'hydrogène ou un groupe carbobenzoxy, par un acide de Lewis pour former la thréo-3-(3,4-dihydroxyphényl)-sérine ou un dérivé N-carbobenzoxy de celle-ci représentés par la formule

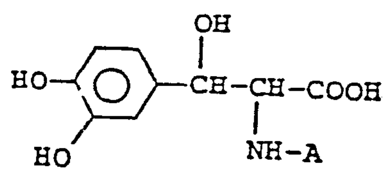

[2],

dans laquelle A a la signification donnée ci-dessus et, si A est un groupe carbobenzoxy, à réduire catalytiquement le composé résultant de formule (2).

2. Procédé selon la revendication 1, dans lequel l'un au moins des composés représentés par les formules (1), (2) et (3) est un composé optiquement actif.

3. Procédé selon la revendication 1, dans lequel A dans les formules (2) et (3) est un atome d'hydrogène.

4. Procédé selon la revendication 1, dans lequel A dans les formules (2) et (3) est un groupe carbobenzoxy.

5. Procédé selon la revendication 4, dans lequel l'un au moins des composés représentés par les formules (1), (2) et (3) est un composé optiquement actif.

6. Procédé pour la préparation d'une thréo-3-(3,4-dihydroxyphényl)-sérine optiquement active, représentée par la formule

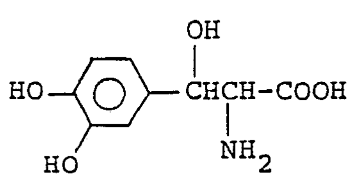

[1],

consistant à faire réagir la thréo-N-carbobenzoxy-3-(3,4-méthylène-dioxyphényl)-serine racémique avec une amine optiquement active, choisie parmi l'éphédrine, la quinidine, la quinine et le 2-amino-1,1-diphénylpropanol pour former un mélange de sels aminés de D- et L-thréo-N-carbobenzoxy-3-(3,4-méthylène-dioxyphényl)-sérine, à séparer le mélange en sel d'amine de D-thréo-N-carbobenzoxy-3-(3,4-méthylène-dioxyphényl)-sérine et en sel d'amine de L-thréo-N-carbobenzoxy-3-(3,4-méthylène-dioxy-phényl)-sérine en mettant à profit la différence de solubilité, à faire réagir chaque sel d'amine avec un acide pour obtenir une (D- ou L-)-thréo-N-carbobenzoxy-3-(3,4-méthylène-dioxyphényl)-sérine optiquement active, à faire réagir le composé optiquement actif résultant avec un acide de Lewis pour obtenir une thréo-N-carbobenzoxy-3-(3,4-dihydroxyphényl)-sérine optiquement active et à réduire catalytiquement le composé résultant.

7. Procédé pour la préparation d'une thréo-3-(3,4-dihydroxyphényl)-sérine optiquement active, représentée par la formule

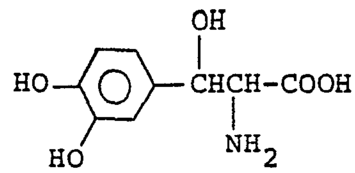

[1],

consistant à traiter la thréo-N-carbobenzoxy-3-(3,4-méthylènedioxyphényl)-sérine racémique par un acide de Lewis pour obtenir la thréo-N-carbobenzoxy-3-(3,4-dihydroxyphényl)-sérine racémique, à faire réagir le composé résultant avec une amine optiquement active choisie parmi la cinchonidine, la brucine, l'éphédrine et le 2-amino-1,1-diphénylpropanol, pour convertir le mélange racémique en un mélange de sels d'amine de D- et de L-thréo-N-carbobenzoxy-3-(3,4-dihydroxyphényl)-sérines, à séparer le mélange en sel d'amine de D-thréo-N-carbobenzoxy-3-(3,4-dihydroxyphényl)-sérine et en sel d'amine de L-thréo-N-carbobenzoxy-3-(3,4-dihydroxyphényl)-sérine en mettant à profit la différence de solubilité, à faire réagir chaque sel d'amine avec un acide pour obtenir une thréo-N-carbobenzoxy-3-(3,4-dihydroxyphényl)-sérine optiquement active, et à réduire catalytiquement le composé optiquement actif résultant.

8. Procédé pour la préparation d'une thréo-3-(3,4-dihydroxyphényl)-sérine optiquement active, représentée par la formule

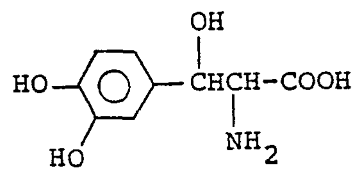

[1],

consistant à faire réagir la glycine avec le pipéronal en présence d'une base, à ajouter de l'acide acétique au mélange réactionnel pour obtenir l'acétate de la thréo-3-(3,4-méthylènedioxyphényl)-sérine racémique représentée par la formule

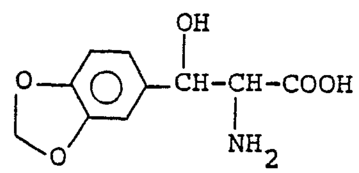

[3'],

à faire réagir cet acétate avec le carbobenzoxychlorure pour obtenir la thréo-N-carbobenzoxy-3-(3,4-méthylene-dioxyphényl)-sérine racémique représentée par la formule

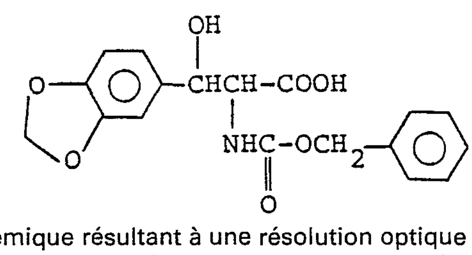

[3"]

à soumettre le mélange racémique résultant à une résolution optique en utilisant une amine optiquement active choisie parmi l'éphédrine, al quinidine, la quinine et le 2-amino-1,1-diphénylpropanol pour obtenir une thréo-N-carbobenzoxy-3-(3,4-méthylène-dioxyphényl)-sérine optiquement active, à traiter le composé optiquement actif résultant par un acide de Lewis pour convertir celui-ci en thréo-N-carbobenzoxy-3-(3,4-dihydroxyphényl-sérine optiquement active représentée par la formule

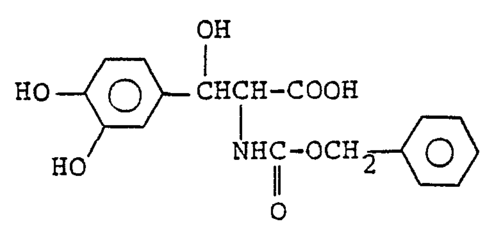

[2']

et à réduire catalytiquement le composé optiquement actif résultant.

9. Procédé pour la préparation d'une thréo-3-(3,4-dihydroxyphényl)-sérine optiquement active, représentée par la formule

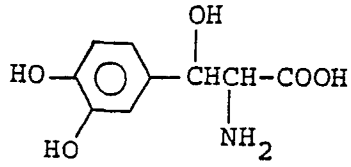

[1],

consistant à faire réagir la glycine avec le pipéronal en présence d'une base, à ajouter de l'acide acétique au mélange réactionnel pour obtenir l'acétate de thréo-3-(3,4-méthylène-dioxyphényl)-sérine racémique représentée par la formule

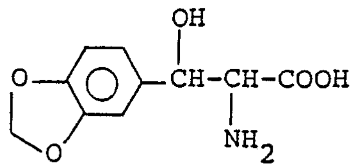

[3'],

à faire réagir cet acétate avec le carbobenzoxychlorure pour obtenir la thréo-N-carbobenzoxy-3-(3,4-méthylène-dioxyphényl)-sérine racémique représentée par la formule

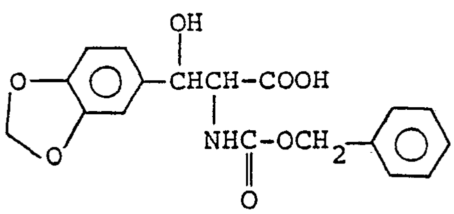

[3"]

à traiter le composé résultant par un acide de Lewis pour obtenir la thréo-N-carbobenzoxy-3-(3,4-dihydroxyphényl)-sérine racémique, à soumettre le mélange racémique résultant à une résolution optique en utilisant une amine optiquement active choisie parmi la cinchonidine, la brucine, l'éphédrine et le 2-amino-1,1-diphénylpropanol pour obtenir une thréo-N-carbobenzoxy-3-(3,4-dihydroxyphényl)-sérine optiquement active et à réduire catalytiquement le composé optiquement actif résultant.

10. Procédé pour la préparation de thréo-3-(3,4-dihydroxyphényl)-sérine racémique ou optiquement active représentée par la formule

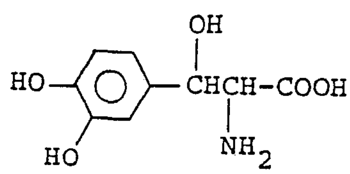

[1],

consistant à faire réagir la glycine avec le pipéronal en présence d'une base, à ajouter de l'acide acétique au mélange réactionnel pour obtenir l'acétate de la thréo-3-(3,4-méthylènedioxyphényl)-sérine racémique représentée par la formule

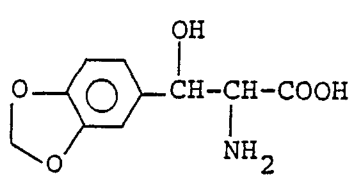

[3'],

et à traiter cet acétate, soit directement, soit après sa résolution en isomères optiquement actifs, par un acide de Lewis.

11. Procédé pour la préparation d'une thréo-3-(3,4-dihydroxyphényl)-sérine optiquement active, consistant à permettre aux cristaux d'une thréo-3-(3,4-dihydroxyphényl)-sérine optiquement active d'être présents dans une solution sursaturée de thréo-3-(3,4-dihydroxyphényl)-sérine racémique et à permettre

que cette thréo-3-(3,4-dihydroxyphényl)-sérine optiquement active sous forme desdits cristaux se sépare préférentiellement à l'état cristallin de la solution sursaturée.

12. Thréo-N-carbobenzoxy-3-(3,4-méthylène-dioxyphényl)-sérine représentée par la formule

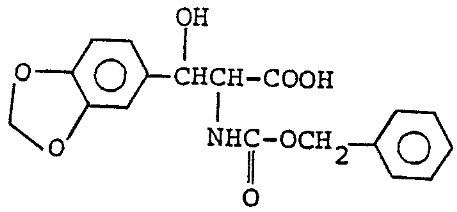

[3"]

13. Thréo-N-carbobenzoxy-3-(3,4-méthylène-dioxyphényl)-sérine optiquement active, représentée par la formule

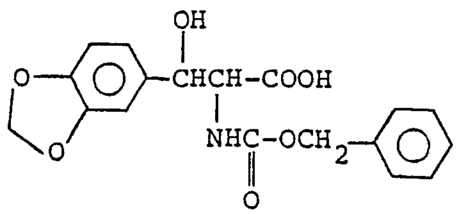

[3"]

14. Procédé pour la préparation d'une thréo-N-carbobenzoxy-3-(3,4-méthylène-dioxyphényl)-sérine représentée par la formule

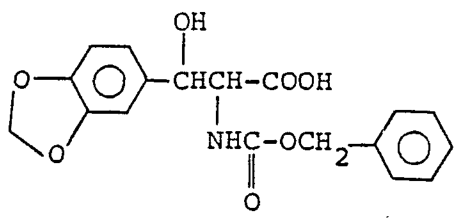

[3"]

consistant à faire réagir la glycine avec le pipéronal en présence d'une base, à ajouter de l'acide acétique au mélange réactionnel pour obtenir l'acétate de thréo-3-(3,4-méthylène-dioxyphényl)-sérine racémique, à faire réagir cet acétate avec le carbobenzoxychlorure et, si nécessaire, à soumettre la thréo-N-carbobenzoxy-3-(3,4-méthylène-dioxyphényl)-sérine racémique résultante à une résolution optique en passant par la formation de sels avec une amine optiquement active.